(19) Europäisches Patentamt / European Patent Office / Office européen des brevets

(11) **EP 1 874 450 B1**

(12) **EUROPÄISCHE PATENTSCHRIFT**

(45) Veröffentlichungstag und Bekanntmachung des Hinweises auf die Patenterteilung:
**08.09.2010   Patentblatt 2010/36**

(21) Anmeldenummer: **06742646.0**

(22) Anmeldetag: **18.04.2006**

(51) Int Cl.:
**B01J 2/06** ^(2006.01)

(86) Internationale Anmeldenummer:
**PCT/EP2006/003721**

(87) Internationale Veröffentlichungsnummer:
**WO 2006/111417 (26.10.2006 Gazette 2006/43)**

(54) **KERAMIKPARTIKEL**

CERAMIC PARTICLES

PARTICULES CERAMIQUES

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **19.12.2005   AT 20262005**
                **18.04.2005   DE 102005018949**

(43) Veröffentlichungstag der Anmeldung:
**09.01.2008   Patentblatt 2008/02**

(60) Teilanmeldung:
**10003178.0 / 2 204 231**

(73) Patentinhaber: **Treibacher Industrie AG
9330 Treibach-Althofen (AT)**

(72) Erfinder:
• **COUFAL, Gerhard
  A-4060 Leonding (AT)**
• **MUSTER, Udo
  A-5023 Salzburg (AT)**

(74) Vertreter: **Schwarz, Albin
  Schwarz & Partner
  Patentanwälte
  Wipplingerstraße 30
  1010 Wien (AT)**

(56) Entgegenhaltungen:
WO-A-01/26794        DE-A1- 4 118 752
DE-A1- 4 338 212     DE-A1- 10 012 551
DE-B- 1 228 231      FR-A- 2 756 196
US-A- 3 578 433      US-B1- 6 174 466

**Beschreibung**

Die vorliegende Erfindung betrifft Partikel aus einem keramischen Werkstoff.

Die US 4,436,782 betrifft die Granulierung eines oligomeren Polyethylenterephthalates zu Pellets.

Aus der DE-OS 100 19 508 A1 ist ein Verfahren und eine Vorrichtung zur schmelzflüssigen Vertropfung von Vorprodukten thermoplastischer Polyester und Copolyester bekannt.

Zerstäubung- und Sprühverfahren sind heutzutage die dominierenden Verfahren zur Herstellung von sphärischen Mikropartikeln. Bei allen diesen Verfahren wird ein Partikelkollektiv mit einer nachteilig sehr breiten Durchmesser-, Masse- und Dichteverteilung erhalten. Überdies zeigen die erzeugten Partikel meist eine geringe Rundheit bzw. Sphärizität. Außerdem können bei der Versprühung und insbesondere der Zerstäubung einerseits nur sehr kleine Partikel und andererseits in ihrer Form und Größe nur sehr unterschiedliche Partikel mit diesen Verfahren erzeugt werden.

Weitere Verfahren des Standes der Technik zur Herstellung sphärischer Partikel sind Granulationsverfahren. Dabei werden beispielsweise keramische Oxide mit einem keramischen Bindemittel vermengt und in klassischen Granulationsverfahren, z.B. mittels Aufbaugranulatoren zu runden Partikeln verformt (z.B. EP 26 918, EP 1136464 A2). Größere Partikel von ca. 3-10 mm werden durch Pressverfahren in Gummimatrizen hergestellt.

Sphärische Partikel aus stabilisierten Zirkonoxiden mit einem $CeO_2$-Gehalt von weniger als 30 Masse-% werden seit kurzem technisch als Mahlkörper eingesetzt und dienen aufgrund ihrer hervorragenden Werkstoffeigenschaften als wirtschaftlich interessante Alternativwerkstoffe zu bekannten stabilisierten Zirkonoxiden des CaO, MgO oder $Y_2O_3$. Beim Einsatz der sphärischen Mahlkörper in modernen Hochleistungsrührwerkskugelmühlen der Nasszerkleinerung ist eine enge Durchmesser-, Masse- und Dichteverteilung technisch von Vorteil.

Gerade bei der Nasszerkleinerung mit modernen Hochleistungsmühlen werden immer höhere Umfangsgeschwindigkeiten und folgend spezifische Energieleistungen vom Rührorgan auf den Mahlkörper übertragen. Der Einsatz dieser Mühlentechnologien erlaubt die Vermahlung der Produkte in den Submikron- und Nanometerbereich. Umgekehrt sind aber entsprechende qualitative Voraussetzungen an die Mahlkörper zu stellen, die sich in sehr einheitlichen und hochdichten Materialien mit sehr engen Durchmesser-, Dichte- und Masseverteilungen finden, da dadurch eine sehr homogene Kraftübertragung vom Mahlkörper auf das Mahlgut bewerkstelligt werden kann und somit die Mahlergebnisse in puncto Partikelfeinheit und Partikelverteilung des Mahlgutes sowie in puncto Verschleiß der Mühle und des Mahlkörpers wesentlich verbessert werden können.

Ein bekanntes Verfahren zur Herstellung von sphärischen Mikropartikeln als Mahlkörper sind z.B. Tropfverfahren. Bei diesen wird zur Herstellung von magnesiumstabilisierten Zirkonoxiden als Mahlkörper im Formgebungsschritt eine wässerige Suspension der Oxide, die mit einem keramischen Bindemittel versetzt wurde, durch eine Düse tropfenweise in eine chemisch härtende Lösung vertropft. In der EP 0 677 325 A1 wird das Abtropfen einer wässerigen Suspension der Oxide $ZrO_2$ und $Mg(OH)_2$ mit einem keramischen Bindemittel in eine chemisch härtende Ionentauscherlösung beschrieben. In der DE 102 17 138 A1 wird ein Zertropfungsverfahren für Actinoidenoxide beschrieben.

Es ist daher Aufgabe der vorliegenden Erfindung, Feststoffpartikel aus einem keramischen Werkstoff bereitzustellen, welche eine hohe Sphärizität (Partikelform) und enge Korngrößen-. Masse- und Dichteverteilung aufweisen. Ferner ist es die Aufgabe, keramische Partikel mit besonderen Eigenschaften zu erzeugen.

Dabei wird eine Erstarrungsflüssigkeit gewählt, woher es vorteilhaft ist, wenn eine strömende Erstarrungsflüssigkeit verwendet wird. Vorteilhaft ist es, wenn die Oberflächenspannung der Erstarrungsflüssigkeit kleiner ist als jene des Ausgangsstoffes, dies bedeutet $\sigma_{Erstarrungsflüssigkeit} < \sigma_{Tropfen, fließfähiger Ausgangsstoff}$. Besonders eine Oberflächenspannung der Erstarrungsflüssigkeit von weniger als 50 mN/m, insbesondere von weniger als 30 mN/m sichert einen Übergang der Tropfen des fließfähigen Ausgangsstoffes in die Erstarrungsflüssigkeit, bei dem Schädigungen oder sogar Zerstörungen der Tropfen beim Phasenübergang vermieden werden.

Ferner ist es vorteilhaft, wenn zwischen Erstarrungsflüssigkeit und dem fließfähigen Ausgangsstoff eine möglichst große Polaritätsdifferenz besteht, die über die Grenzflächenspannung definierbar ist. Vorteilhaft sind Grenzflächenspannungen zwischen 25 und 50 mN/m, insbesondere zwischen 30 mN/m, ganz besonders zwischen 35 und 50 mN/m.

Als Ausganssstoff kann eine fließfähige Suspension eingesetzt werden, die einen keramischen Werkstoff und ein Bindemittel enthält und welche zur Verfestigung in eine strömende oder auch nicht-strömende, insbesondere bei nicht-strömenden in eine ruhende Erstarrungsflüssigkeit, in der eine chemische Härtung hervorgerufen wird, eingebracht wird.

Für die Herstellung von Feststoffpartikeln mit hoher Sphärizität ist ein entsprechend hoher Polaritätsunterschied, charakterisiert durch eine entsprechend hohe Grenzflächenspannung zwischen den Tropfen des fließfähige Ausgangsstoffes und der Erstarrungsflüssigkeit in Kombination mit einer gezielt eingestellten Verfestigung der erzeugten Tropfen des fließfähige Ausgangsstoffes zu den Feststoffpartikeln vorteilhaft.

Hierbei sind die Grenzflächenspannung bzw. der Polaritätsunterschied wie folgt definiert:

Als Maß für die Größe des Polaritätsunterschieds - zwischen dem fließfähigen Ausgangsstoff und der Erstarrungsflüssigkeit- wird die Grenzflächenspannung verwendet. Da die Werte für die Grenzflächenspannung experimentell sehr schwer zugänglich sind, wird sie über die einerseits experimentell leicht zugänglichen und andererseits in der einschlägigen Literatur ausreichend gut dokumentierten Oberflächenspannungen bestimmt. Hierzu wird die Oberflächenspannung einer Mediumsphase ($\sigma$) als Summe der unpolaren Wechselwirkungen ($\sigma_D$, London-Dispersion-Kräfte) und der polaren Wechselwirkungen ($\sigma_P$, polare Kräfte) beschrieben. Der Index i bezieht sich auf die jeweilige Phase bzw. der Index ij auf eine Phasengrenze.

$$\sigma_i = \sigma_{D,i} + \sigma_{P,i}$$

$\sigma_i$      Oberflächenspannung der Mediumsphase i [mN/m]

$\sigma_{D,i}$      Unpolarer Anteil der Oberflächenspannung, London-Dispersionsanteil [mN/m]

$\sigma_{p,i}$      Polarer Anteil der Oberflächenspannung [mN/m]

Experimentell werden die unpolaren und polaren Anteile der Oberflächenspannung über die Kontaktwinkelmethode bestimmt. So zeigt beispielsweise Wasser bei 20 °C eine Oberflächenspannung ($\sigma_{Wasser}$) von 72,8 mN/m mit einem unpolaren Anteil von $\sigma_{D,Wasser}$ von 21,8 mN/m und einem polaren Anteil von $\sigma_{P,Wasser}$ von 51,0 mN/m. Mit der Kenntnis der polaren und unpolaren Anteile der Oberflächenspannungen ist die Grenzflächenspannung zwischen 2 Mediumsphasen wie folgt definiert:

$$\sigma_{ij} = \sigma_i + \sigma_j - 2 * (\sqrt{\sigma_{D,i} * \sigma_{D,j}} + \sqrt{\sigma_{P,i} * \sigma_{P,j}})$$

$\sigma_{ij}$      Grenzflächenspannung der Mediumsphasen i und j an der Phasengrenze, z.B. fließfähiger Ausgangsstoff und Erstarrungsflüssigkeit [mN/m]

$\sigma_{D,i}, \sigma_{D,j}$      Unpolarer Anteil der Oberflächenspannung der Mediumsphasen i und j [mN/m]

$\sigma_{p,i}, \sigma_{p,j}$      Polarer Anteil der Oberflächenspannung der Mediumsphasen i und j [mN/m]

Allgemein gilt, dass bei einem hohen Wert der Grenzflächenspannung ein großer Polaritätsunterschied zwischen den beiden Mediumsphasen besteht. Die Oberflächen- und/oder Grenzflächenspannungen sind temperaturabhängig und werden insofern auf eine Temperatur von 20 °C. per Definition bezogen.

Der Polaritätsunterschied zwischen dem fließfähigen Ausgangsstoff und der Erstarrungsflüssigkeit kann alternativ auch mit dem Kontaktwinkel $\varphi$ zwischen zwei Fluidphasen oder dem Benetzungswinkel zwischen einer Fluid- und Feststoffphase beschrieben werden.

$$\cos \varphi = \frac{\sigma_i - \sigma_{ij}}{\sigma_j}$$

$\sigma_{ij}$      Grenzflächenspannung der Mediumsphasen i und j an der Phasengrenze, z.B. fließfähiger Ausgangsstoff und Esstarrungsflüssigkeit [mN/m]

$\sigma_i$      Oberflächenspannung der Mediumsphasen i, Erstarrungsflüssigkeit [mN/m]

$\sigma_j$      Oberflächenspannung der Mediumsphasen j, fließfähiger Ausgangsstoff [mN/m]

Aufgrund der entgegen gesetzten Wechselwirkungen bzw. bei einem entsprechend hohen Polaritätsunterschied von Tropfen des fließfähigen Ausgangsstoffes und der Erstarrungsflüssigkeit bildet sich unterstützend die kleinste Phasengrenze zwischen den beiden Mediumsphasen aus. Dies ist eine Kugeloberfläche, besonders wenn der abgetauchte Tropfen noch über eine ausreichend kurze Zeitspanne fließfähig bleibt, insbesondere bei Tropfen aus Schmelze, ganz besonders bei Harnstoff-enthaltenden Schmelzetropfen. Hierbei entsteht, aufgrund der freiwerdenden Kristallisationswärme, ein Wärmestrom in Richtung Phasengrenze bzw. in Richtung des Temperaturgradienten. Der Ausgangstropfen bleibt bei der charakteristischen Umwandlungstemperatur (Abführung einer latenten Wärme) zunächst ausreichend fließfähig, so dass vorteilhaft eine Reformation des eventuell geschädigten Partikels zum sphärischen Partikel bewirkt werden kann.

Bei der Zertropfung eines fließfähigen Ausgangsstoffes auf Basis eines keramischen Werkstoffes und eines Bindemittels kann der Polaritätsunterschied zwischen der Suspension und der Erstarrungsflüssigkeit vorteilhaft genutzt werden, insbesondere wenn die Erstarrungsflüssigkeit aus zwei wenig oder nicht miteinander mischbaren Phasen bzw.

Polaritäten und/oder unterschiedlichen Dichten besteht, so dass insbesondere die unpolare, weniger dichte und oberflächenniedrigere Phase gegenüber dem fließfähigen Ausgangsstoff den noch fließfähigen Partikel zu einem sphärischen Partikel formt bzw. reformiert, und nachfolgend in der dichteren Phase die chemische Härtung bewirkt wird.

Besonders vorteilhaft bei der Zertropfung eines fließfähigen Ausgangsstoffes auf Basis eines keramischen Werkstoffes und eines Bindemittels ist weiterhin der Einsatz einer Erstarrungsflüssigkeit, die aus zumindest zwei mischbaren Komponenten unterschiedlicher Polarität besteht, wobei durch die weniger polare Komponente die entgegen gesetzte Wechselwirkung für die Bildung eines sphärischen Partikels genutzt wird und durch die Herabsetzung der Reaktionsgeschwindigkeit durch die weniger polare Komponente die chemische Härtungszeit angehoben werden kann, so dass der zu einem sphärischen Partikel reformierende Partikel über eine ausreichende Zeitspanne fließfähig bleibt und entsprechend gezielt chemisch gehärtet wird.

Ganz besonders vorteilhaft bei der Zertropfung eines fließfähigen Ausgangsstoffe auf Basis eines keramischen Werkstoffes und eines Bindemittels ist die Kombination einer Erstarrungsflüssigkeit, bestehend aus zwei nicht mischbaren Phasen bzw. Polaritäten und/oder unterschiedlicher Dichten, so dass insbesondere die unpolare, weniger dichte und oberflächenniedrigere Phase gegenüber dem fließfähigen Ausgangsstoff den Partikel zu einem sphärischen Partikel formt bzw. reformiert, da dieser noch ausreichend fließfähig ist, und in der dichteren Phase die chemische Härtung durch den Zusatz einer mischbaren aber weniger polaren Komponente die chemische Härtung zeitlich gesteuert werden kann.

In einer Ausführungsform des erfindungsgemäßen Verfahrens wird eine Grenzflächenspannung zwischen den Tropfen des fließfähigen Ausgangsstoffes und der Erstarrungsflüssigkeit zwischen 25 bis 50 mN/m, insbesondere zwischen 30 und 50 mN/m und ganz besonders zwischen 35 und 50 mN/m eingestellt.

Ferner ist bevorzugt, wenn eine Erstarrungsflüssigkeit gewählt wird, so dass der Kontaktwinkel zwischen dem fließfähigen Ausgangsstoff und der Erstarrungsflüssigkeit und/oder der Benetzungswinkel zwischen dem gehärteten Ausgangsstoff und der Erstarrungsflüssigkeit > 45° und besonders bevorzugt > 90° ist.

Als Erstarrungsflüssigkeit wird bei einem polaren fließfähigen Ausgangsstoff, ein unpolares Fluid eingesetzt, insbesondere ein aliphatischer hochsiedender, ein ungesättigter, ein aromatischer, ein cyclischer, ein halogenierter Kohlenwasserstoff und/oder Kohlenwasserstoffe mit mindestens einer Ester-, Keto-, Aldehydgruppierung oder ein Gemisch aus mindestens zwei Kohlenwasserstoffen, insbesondere ein Aliphatengemisch aufweist oder aus ihnen besteht.

Weitere vorteilhafte Ausführungsformen dazu werden zum Gegenstand von Unteransprüchen gemacht und im Zusammenhang mit den Figuren beschrieben.

Die Erfindung wird nachfolgend unter Bezugnahme auf die Figuren der Zeichnungen an mehreren Ausführungsbeispielen näher erläutert. Es zeigen:

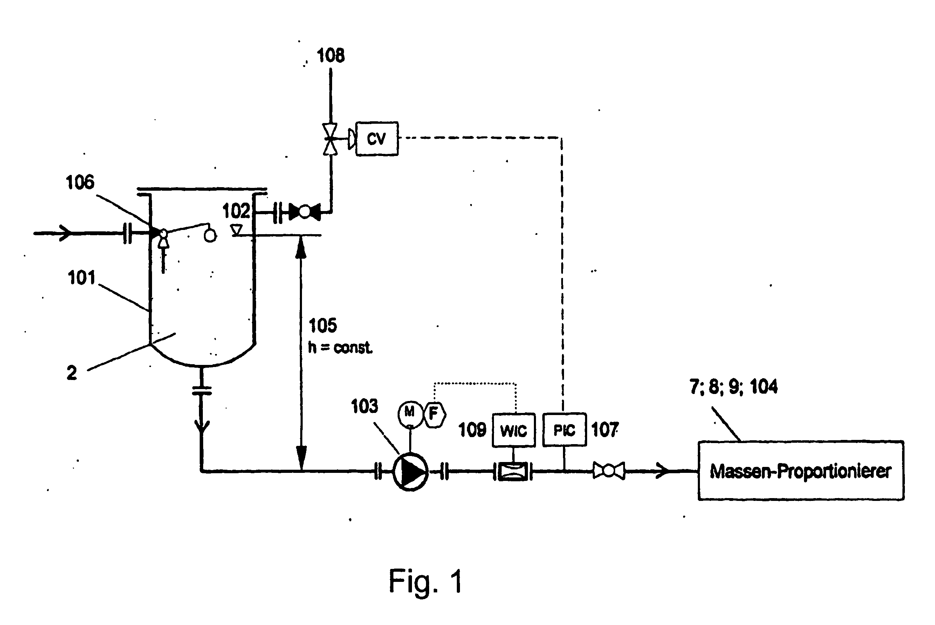

➢ Fig. 1: Verfahrensfließbild für die Steuerung und/oder Regelung eines konstanten Massestroms einer Ausführungsform des erfindungsgemäß angewendeten Verfahrens und der erfindungsgemäß angewendeten Vorrichtung;

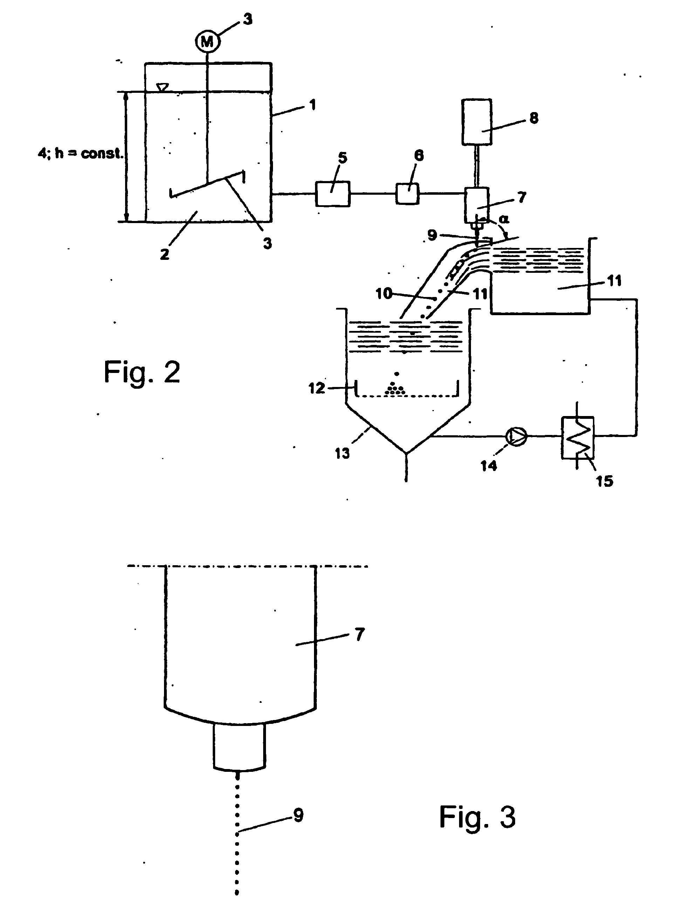

➢ Fig. 2: ein Verfahrensfließbild einer Ausführungsform des erfindungsgemäß angewendeten Verfahrens (Rinnenkanal) und einer erfindungsgemäß angewendeten Vorrichtung;

➢ Fig 3: eine schematische Darstellung eines stehenden Tropfenbildes;

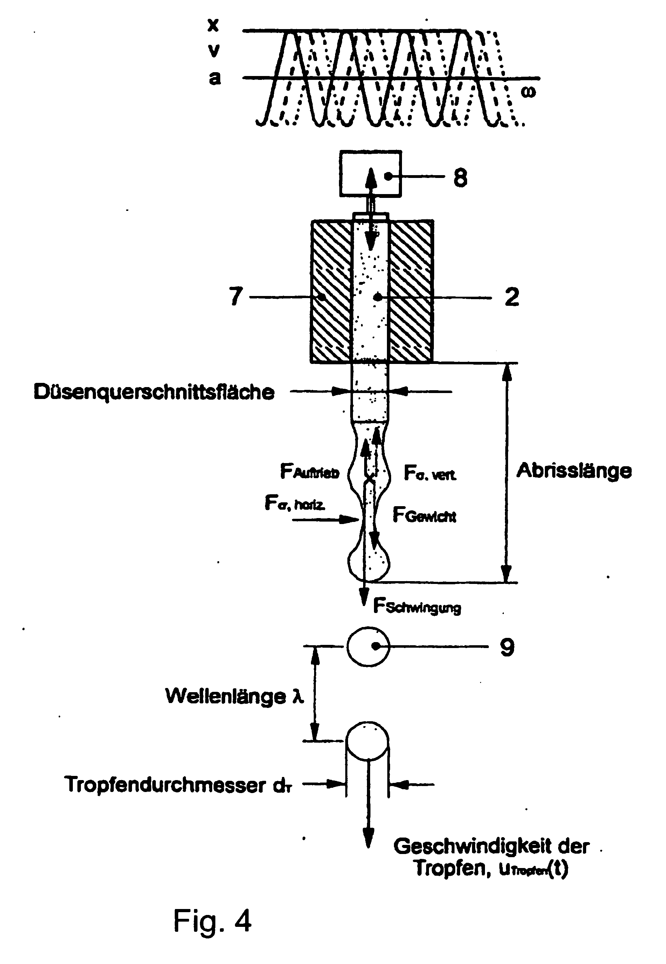

➢ Fig. 4: eine schematische Darstellung der Zertropfung (Masseproportionierer) eines laminaren Strahlzerfalls mit Resonanzanregung des fließfähigen Ausgangsstoffes:

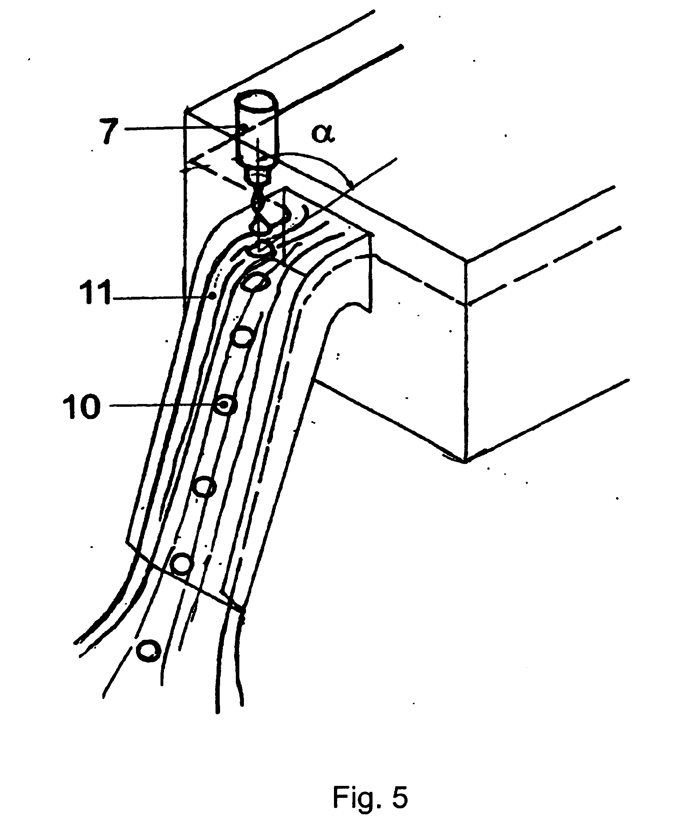

➢ Fig. 5: eine perspektivische Ansicht der Eintropfung gemäß der Ausführungsform des erfindungsgemäß angewendeten Verfahrens gemäß Fig 4 (Rinnenkanal);

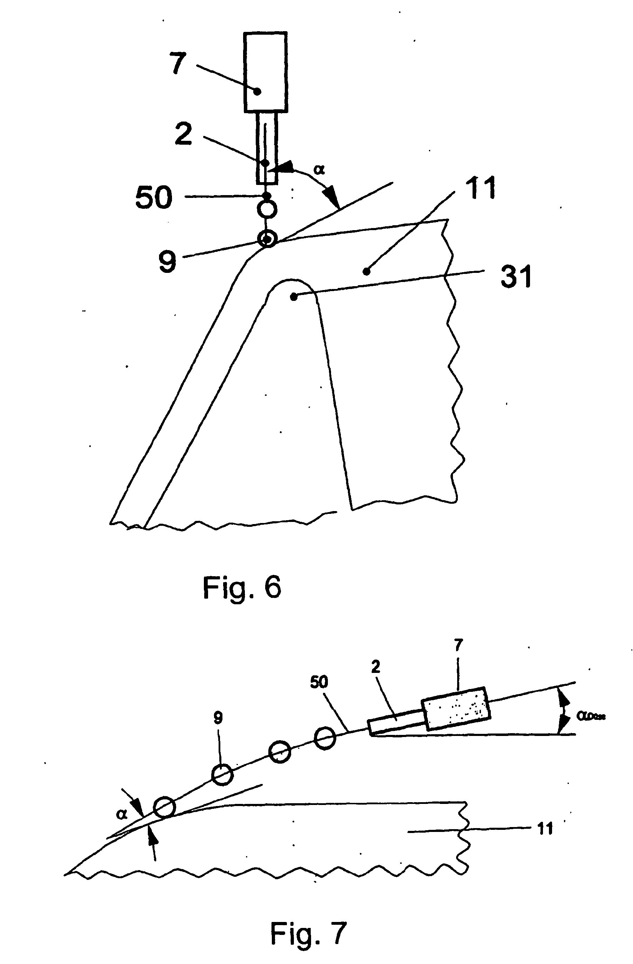

➢ Fig. 6: eine Seitenansicht der Eintropfung gemäß einer Ausführungsform des erfindungsgemäß angewendeten Verfahrens;

➢ Fig. 7: eine schematische Darstellung der Reduktion der Relativgeschwindigkeit durch Veränderung des Auftreffwinkels mittels einer gekrümmten Bewegungsbahn;

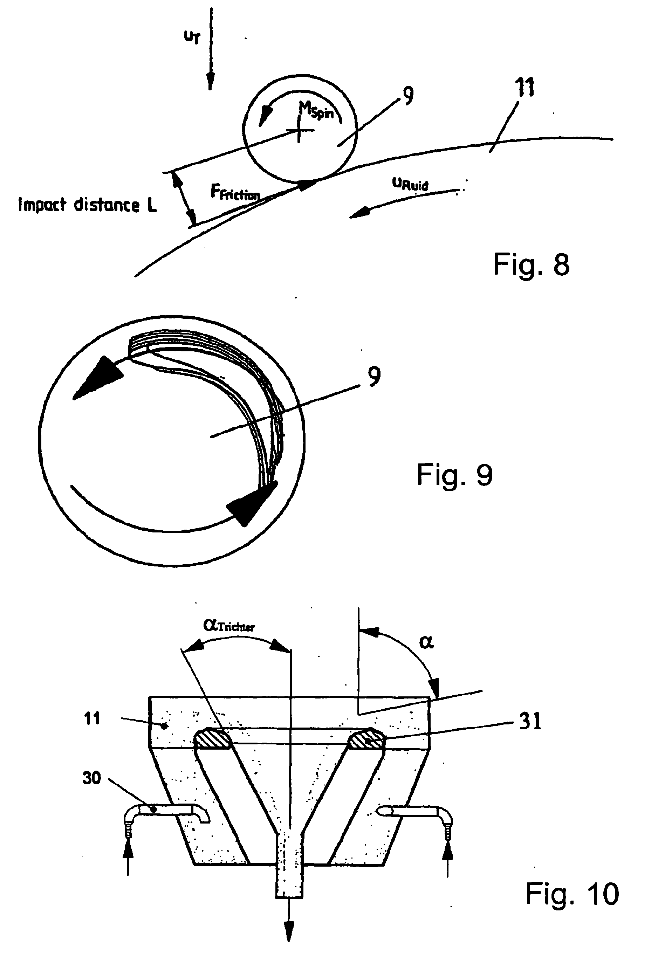

➢ Fig. 8: eine Prinzipskizze betreffend der Erzeugung eines Spins;

➢ Fig. 9: eine räumliche Darstellung einer Kugel, welche durch ein zweidimensionales Geschwindigkeitsfeld eine Rotation erfahren hat - Stabilisierungseffekt;

➢ Fig. 10: eine schematische Darstellung einer Ausführungsform der erfindungsgemäß verwendeten Vorrichtung (Rinnenkanaltrichter mit Überlaufkante);

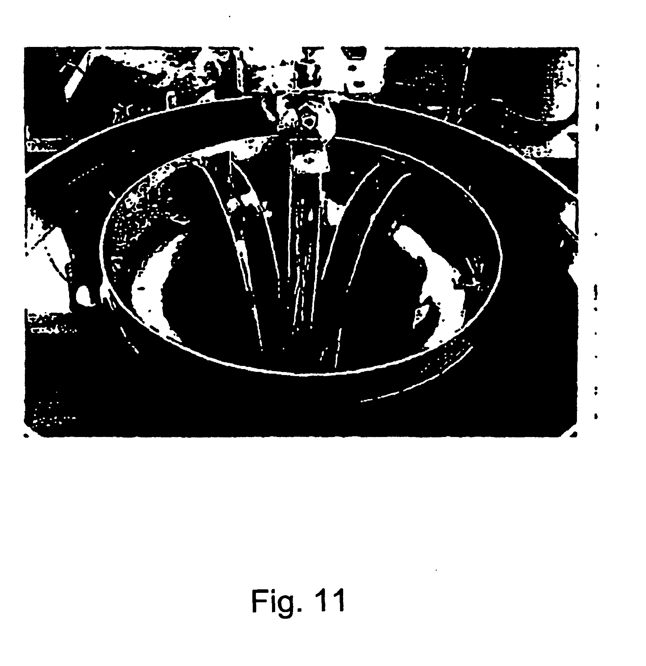

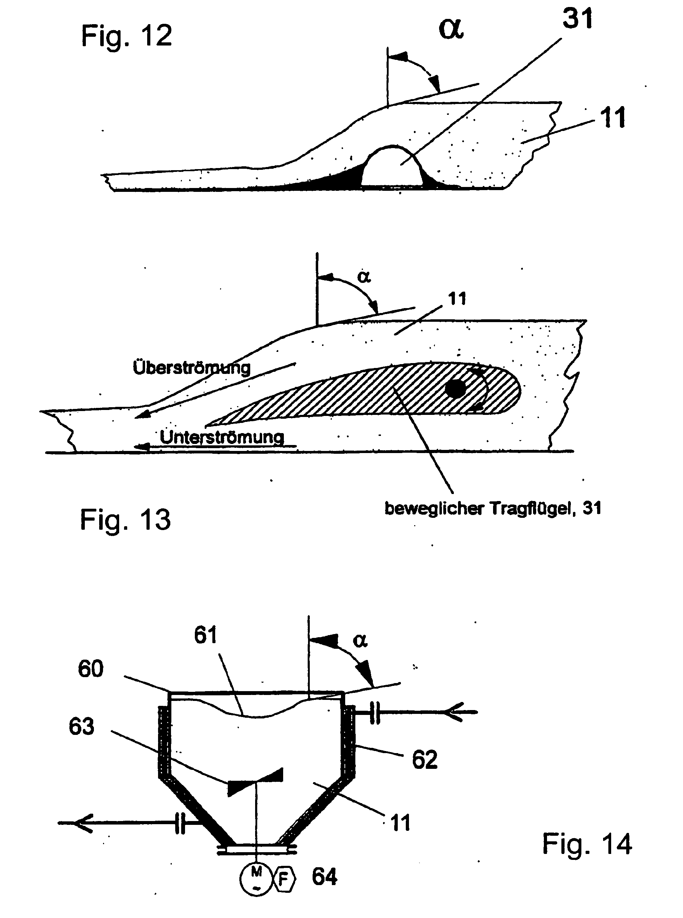

➢ Fig. 11: eine fotographische Darstellung eines Rinnentrichters einer vorteilhaften Ausgestaltung der erfindungsgemäß angewendeten Vorrichtung gemäß Fig. 13 (Rinnenkanaltrichter mit Überlaufkante, 3 Rinnen);

➢ Fig. 12: eine Schnittansicht einer alternativen Ausführungsform einer erfindungsgemäß angewendeten Vorrichtung (Rinnenkanal mit Strömungsstörkörper);

➢ Fig. 13: eine Schnittansicht einer alternativen Ausführungsform einer erfindungsgemäß angewendeten Vorrichtung (Rinnenkanal mit verstellbaren Strömungsstörkörper);

➢ Fig. 14: eine Schnittansicht einer Ausführungsform der erfindungsgemäß angewendeten Vorrichtung unter Verwendung einer Rotationsstrümung in Form einer Trombe;

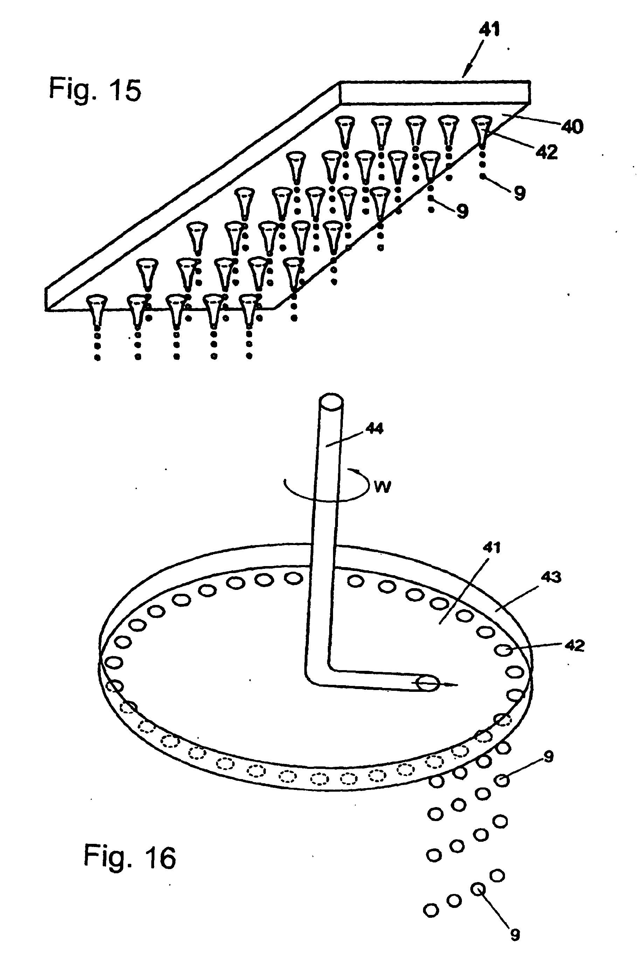

➢ Fig. 15: eine schematische perspektivische Ansicht einer Lochplatte als Abtropfvorrichtung;

➢ Fig. 16: eine_schematische perspektivische Ansicht einer Lochplatte mit rotierender Zufuhr des fließfähigen Ausgangsstoffes zum Abtropfen;

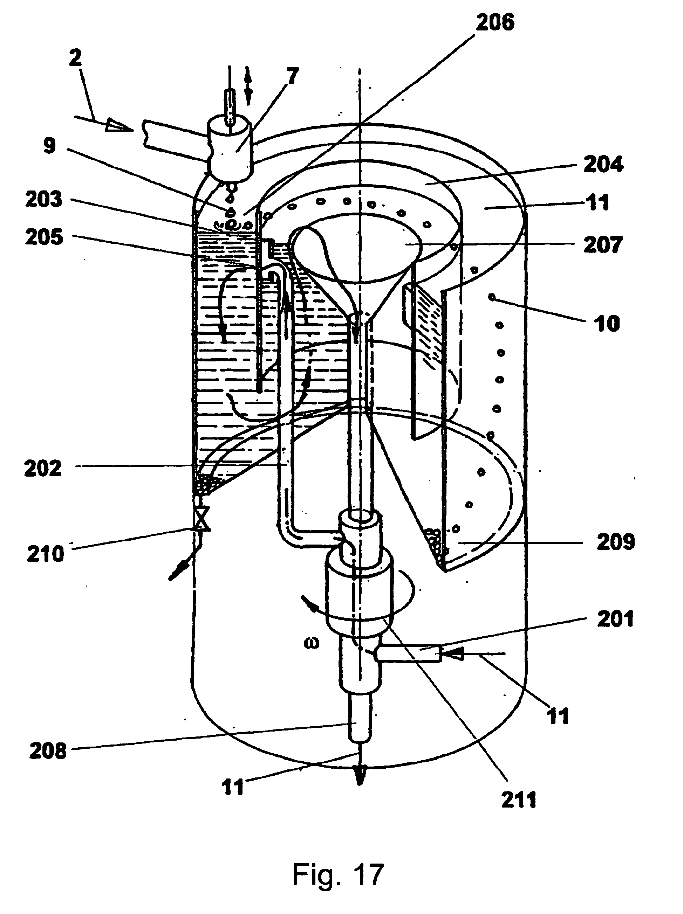

➢ Fig. 17: eine perspektivische Darstellung einer bevorzugten Ausführungsform des erfindungsgemäß angewendeten Verfahrens (rotierender Behälter);

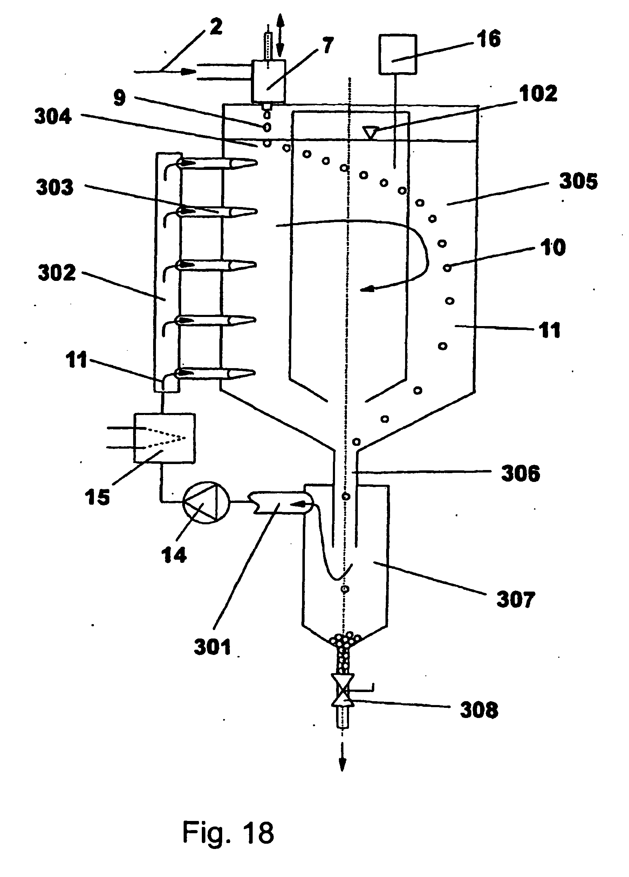

➢ Fig. 18: eine Seitenansicht einer bevorzugten Ausführungsform des erfindungsgemäß angewendeten Verfahrens (Drallbewegung im stationären Ringkanalbehälter durch tangentiale Einleitung der Erstarrungsflüssigkeit);

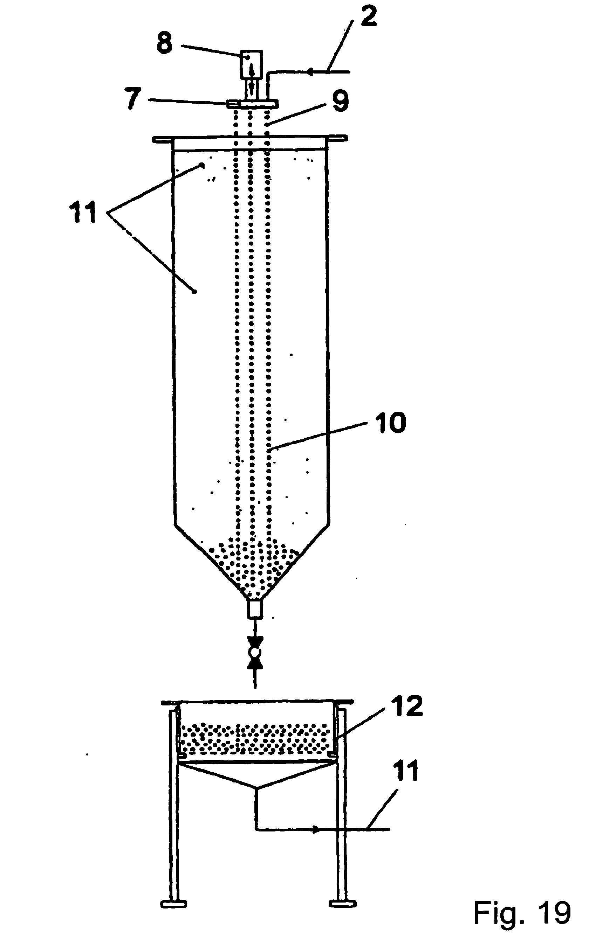

➢ Fig. 19: eine schematische Darstellung einer besonderen Ausführungsform des erfindungsgemäß angewendeten Verfahrens für die Herstellung von sphärischen Feststoffpartikeln auf Basis keramischer Werkstoffe durch Verwendung von zwei nicht miteinander mischbaren Phasen der Erstarrungsflüssigkeit.

Grundsätzlich gibt es unterschiedliche und bekannte Methoden einen fließfähigen Ausgangsstoff in einzelne Tropfen zu zerteilen. Beim Ausfließen des fließfähigen Ausgangsstoffes durch eine Düse, Kappillare oder Lochplatte bildet die Flüssigkeit zunächst einen Strahl, der durch Instationäritäten in einzelne Tropfen zerfällt. Je nach dem beim Strahlzerfall vorherrschenden Strömungsregime unterscheidet man:

➢ Abtropfen
➢ laminarer Strahlzerfall (Zertropfen)
➢ Zerwellen
➢ turbulenter Strahlzerfall (Zerstäuben, Versprühen)

Für die Erzielung von Partikel mit möglichst engen Korngrößen-, Masse- und Dichteverteilungen sind insbesondere die Strömungsregime des Abtropfens und des laminaren Strahlzerfalls von Interesse. Beim Abtropfen gehen die Ausströmgeschwindigkeiten gegen Null und die Strömungs- und Reibungskräfte sind vernachlässigbar klein.

Steigert man die Strömungsgeschwindigkeit, bildet sich über einem mittels der Reynoldszahl [Re] definierbaren Strömungsbereich ein laminarer Strahl aus. Die kritische-Strahl-Reynoldszahl [$Re_{krit,Strahl}$] definiert den Übergang von laminaren zu turbulenten Strömungsverhältnissen bzw. grenzt die beiden Strömungsregime zueinander ab. Die $Re_{krit,\,Strahl}$ ist eine Funktion der dimensionslosen Kennzahl Ohnesorge [Oh] und grenzt über eine bekannte Ungleichungsbeziehung den kapillaren Zerfall (laminar) von dem durch aerodynamische Kräfte beeinflussten Zerfall (turbulent) ab. Es wird festgehalten, dass die $Re_{krit,Strahl}$ einerseits von den Stoffeigenschaften des zu zertropfenden Fluids (fließfähiger Ausgangsstoff) und andererseits vom eingesetzten Düsen- bzw. Lochdurchmesser definiert wird und im Unterschied zu ausgeprägten Rohrströmungen (z.B. $Re_{krit,Rohr}$ = 2.320) keinen absoluten Wert besitzt.

Instationäritäten führen in der Regel dazu, dass unterschiedlich große Tropfen 9 entstehen. Durch das Aufprägen einer mechanischen Schwingung 8, die auf unterschiedlichste und bekannte Weise erzeugt werden kann, auf die Flüssigkeitssäule, die Kapillare oder die umgebende Luft kann die Bildung von Tropfen gleicher Größe erreicht werden. Durch die periodische Störung wird der Strahl in gleich bleibende Abstände eingeschnürt. Trotz dieser bekannten Vorkehrungen sind die Voraussetzungen zu schaffen, die zu einer Konstanthaltung des Massestromes und seiner Temperatur (Dichte) führen. Es ist verständlich, dass trotz einer konstant gehaltenen periodischen Störung bei einer Schwankung eines laminar geführten Massestromes und seiner Temperatur (Dichte) unterschiedlich große Tropfen 9 erzeugt werden würden.

Für die Erzeugung eng verteilter Korngrößen- und insbesondere Masseverteilungen durch laminaren Strahlzerfall ohne und insbesondere mit Schwingungs- bzw. Resonanzanregung wird der fließfähige Ausgangsstoff 2 unter Zwang zum eigentlichen Masseproportionierer 7, 8 gefördert. In dieser Vorrichtung wird der konstant gehaltenen Massestrom bei konstanter Temperatur (Dichte) unter laminaren Strömungsverhältnissen in eng masseverteilte Tropfen 9, bevorzugt durch Aufbringung einer periodischen Störung, zerlegt. Zwischen dem konstant gehaltenen Massestrom [M] und dem erzeugten Durchmesser der Tropfen [$d_T$], der Anregerfrequenz [f] und der Dichte [$\rho_{Fluid}$] gilt folgende Beziehung:

$$\dot{M} = \left(\frac{d_T^3 * \pi}{6}\right) * \frac{f}{\rho_{Fluid}}$$

M      Massestrom des Fluids [kg/s]
$d_T$      Durchmesser des Tropfens [m]
$\rho_{Fluid}$      Dichte des Fluids, fließfähiger Ausgangsstoff [kg/m³]
f      Frequenz der periodischen Störung [Hz bzw. l/s]

Die Dichte des fließfähigen Ausgangsstoffes bzw. insbesondere des Massestromes ist eine Funktion der Temperatur, deshalb wird der Zertropfungsprozess vorteilhaft unter Kontrolle einer gemessenen, definierten Temperatur geführt. Bei einem konstant gehaltenen Massestrom und einer definierten periodischen Störung der Frequenz f sowie der bekannter, konstanter Temperatur (Dichte $\rho$ und anderer temperaturabhängiger Stoffeigenschaften) wird ein definierter Durchmesser $d_T$ des Tropfens 9 erzeugt.

Die Einstellung eines konstanten Massestromes (siehe Fig. 1) unter Zwangsförderung kann auf verschiedenste Weise bewerkstelligt werden, zum Beispiel

➧ durch ein konstant gehaltene Druckdifferenz, entweder über eine technisch bekannte Druckregelung 107 mittels Druckregelventil CV oder durch eine definierte Überlagerung der Fluidphase des fließfähigen Ausgangsstoffes mit einem Druckgas 108,

➧ durch exakte Einstellung einer hydrostatischen Höhe 105 des fließfähigen Ausgangsstoffes 2 mit Nachspeisung des fließfähigen Ausgangsstoffes 2 unter Konstanthaltung des Fluidniveaus 102 über ein Schwimmerventil 106,

➧ durch eine druckerhöhende Pumpe 103, insbesondere eine pulsationsfrei laufende Pumpe 103.

➧ oder durch Kombinationen der beispielhaft angeführten Varianten.

Der Massestrom wird beispielsweise mit einem Massedurchflussmeßgerät 109 nach dem Coriolis-Messprinzip gemessen, wobei der Messwert auch für die Regelung des Masseflusses durch Drehzahlregelung der Pumpe 103 genutzt wird. Heutzutage kommerziell erhältliche Coriolis-Sensoren besitzen die Vorteile der simultanen Masse-, Dichte-, Temperatur- und Viskositätsmessung, so dass alle für die Kontrolle des Zertropfungsprozesses relevanten Parameter gleichzeitig erfasst und geregelt werden können.

Es hat sich gezeigt, dass die Korngrößenverteilung vorteilhaft verengt werden kann, wenn der fließfähigen Ausgangsstoff zertropft wird, indem ein laminarer Strahl des fließfähigen Ausgangsstoffes 2 einer Resonanzanregung ausgesetzt wird. Im Masseproportionierer 7, 8, 104 wird der laminar und massekonstant geführte Strahl des fließfähigen Ausgangsstoffes, insbesondere durch eine periodische Störung bzw. Störkraft der Frequenz f in massegleiche Tropfen 9 periodisch zerteilt oder periodisch eingeschnürt (siehe Fig. 4). Durch das Aufprägen dieser periodischen bzw. insbesondere dieser harmonischen Schwingung der Frequenz f auf die Flüssigkeitssäule, die Düse (Kapillare, Schwinglochplatte), das umgebende Medium oder durch eine Zerschneidung des Strahles wird die Bildung von Tropfen 9 mit enger Masseverteilung vorteilhaft erzielt. Die Aufprägung einer definierten und periodischen Störkraft auf mechanischem, elektromechanischem und/oder elektromagnetischem Weg kann über ein harmonisches Schwingungssystem (Elektromagnet, Piezokristallsonde, Ultraschallsonde, rotierender Draht, Schneidwerkzeug, Stab) erfolgen. Tropfenzerteiler dieser Bauarten sind an sich bekannt.

Zwischen dem Durchmesser der zu erzeugenden Tropfen ($d_T$), der durch eine periodische Störung eines massedefinierten und laminar geführten Flüssigkeitsstrahles des fließfähigen Ausgangsstoffes (2) mit der Frequenz f erzeugt wird, und dem Durchmesser des Strahles bzw. der Düsenöffnung $D_{Düse}$ kann entsprechend der bekannten Beziehungen von Lord Rayleigh und Weber über die dimensionslosen Kennzahlen, insbesondere ka (Wellenzahl) und/oder $ka_{opt,Raylcigh}$ (optimale Wellenzahl nach Rayleigh) und/oder $ka_{opt, Weber}$ (optimale Wellenzahl nach Weber) eine optimale Anregungsfrequenz für das jeweilig betrachtete Stoffsystem ermittelt und definiert werden. Entsprechend dieser Berechnungen zeigt sich ein entsprechender stabiler Arbeitsbereich der Zertropfung. Die Gültigkeit dieser Gesetzmäßigkeiten des laminaren Strahlzerfalls mit Resonanzanregung, bei der Zertropfung von Suspensionen eines keramischen Werkstoffs auf Basis $CeO_2/ZrO_2$ mit einem Bindemittel, können über die dimensionslosen Kennzahlen Bond [Bo], Weber [We], Ohnesorge [Oh] und Froude [Fr] bestätigt werden. In diesem ermittelten Arbeitsbereich ist eine Steuer- und Regelbarkeit der Tropfenerzeugung, insbesondere unter der Prämisse eines konstanten Massestromes des fließfähigen Ausgangsstoffes, besonders gut bewerkstelligbar.

In Fig. 2 ist der grundsätzliche Aufbau einer Ausführungsform des erfindungsgemäß angewendeten Verfahrens im Überblick dargestellt. Fig. 4 stellt dann eine besondere Ausgestaltung der Zertropfung im Detail dar.

In Fig. 2 wird der fließfähige und zu zertropfende Ausgangsstoff 2 aus einem Vorratsbehälter 1 zur Masseproportionierungseinheit 7 (mit einer Düse) mit Resonanzanregung 8 gefördert, in der die Zertropfung stattfindet. Der Ausgangsstoff 2 kann zwecks Erzielung einer möglichst homogenen Phase mit einem Rührorgan 3 ständig bewegt werden. In einer vorteilhaften Ausführung wird im Vorratsbehälter 1 ein konstantes Fluidniveau 4 eingestellt, so dass sowohl auf eine installierte Pumpe 5 als auch auf den Masseproportionierer 7 ein quasi konstanter Vordruck wirkt. Die Einstellung des Druckes kann auch über eine entsprechende Druckgasüberlagerung des Fluidniveaus 4 bewerkstelligt werden.

Der Ausgangsstoff 2 wird über eine Pumpe 5 und folgend über einen Massedurchflusszähler 6, der z.B. nach dem Coriolis-Messprinzip arbeitet, gefördert. In diesem Fall wird die Kreiselpumpe 5 über die Führungsgröße Massestrom vorteilhaft drehzahlgeregelt, so dass ein konstanter Massestrom zum Masseproportionierer 7 eingestellt wird.

Der, hier z.B, unter Zwang geförderte und massestromkonstante geförderte, fließfähige Ausgangsstoff 2 wird durch eine Öffnung in Form einer Düse 7, die hier als Teil eines Masseproportionieres aufgefasst wird, unter laminaren Strömungsverhältnissen gedrückt. Dem Strahl des fließfähigen Ausgangsstoffs 2 wird eine harmonische Schwingung (Sinusschwingung) mittels elektronisch geregeltem Elektromagneten 8 überlagert. Die für den Ablösevorgang relevante Beschleunigung a der periodisch eingeleiteten Störkraft ist um die Phase π [rad] gegenüber der Amplitude x der Schwingung verschoben. Der Ausgangsstoff 2 bildet zunächst einen laminar strömenden Strahl aus, der kurz nach der Düsenöffnung 7, aber mit einem entsprechenden Abstand zur Düse, gemäß den Gesetzmäßigkeiten des laminaren Strahlzerfalls zerfällt. Durch die dem Ausgangsstoff 2 aufgeprägte Schwingungskraft wird eine definierte und periodisch wiederkehrende Soll-Bruchstelle im Strahl bewirkt, so dass immer gleich schwere Tropfen 9 (und damit später Partikel) mit einem Tropfendurchmesser $d_T$ entstehen (Mengen- bzw. Masseproportionierung), die noch in sich schwingen. Der treibenden Kraft der Ablösung wird die Schwingungskraft periodisch addiert.

Die Tropfen 9 des fließfähigen Ausgangsstoffes 2 bewegen sich dann entlang einer Bewegungsbahn 50 in Richtung Erstarrungsflüssigkeit 11. Falls keine zusätzlich eingebrachten Kräfte - z.B. aerodynamische Kräfte - auf die Tropfen 9 wirken, fallen die Tropfen der Schwerkraft folgend nach unten.

Diese Anordnung erlaubt die Variation der Herstellung von unterschiedlichen Durchmessern der Feststoffpartikel durch Variation der Schwingungsfrequenz f, der Amplitude x, des Düsendurchmessers $D_{Düse}$ und der Variation des konstant zu haltenden Massestromes. Durch diese Anordnung können somit gezielt definierte Tropfen 9 mit sehr engen Dichte-, Masse- und Durchmesserverteilungen hergestellt werden, ohne dabei die Düsenbohrung verändern zu müssen.

Eine weitere Variationsmöglichkeit besteht in der Veränderung der Stoffeigenschaften beispielsweise durch die Veränderung der Temperatur, wodurch die Stoffeigenschaften Viskosität, Oberflächenspannung und/oder Dichte einem optimalen Vertropfungsbild angepasst werden können.

Eine optimal eingestellte schwingungsüberlagerte Zertropfung des laminaren Strahlzerfalls zeigt sich in einem so genannten stehenden Tropfenbild Fig. 3 welches über eine elektronisch gesteuerte Stroboskoplampe visualisiert werden kann. In diesem Fall entspricht die Tropfenverteilung einer monomodal verteilten Normalverteilung in Bezug auf die Masse

Damit ist an Beispielen beschrieben worden, wie verschiedene masseeng verteilte Tropfen 9 aus einem fließfähigen Ausgangsstoff 2 herstellbar sind. Die beschriebenen Vorrichtungen zur Masseproportionierung werden in einer Anlage verwendet, in der die Tropfen 9 in eine Erstarrungsflüssigkeit 11 eingetropft werden, um feste Feststoffpartikel 10 zu bilden.

Nach dem Zerfall des Strahles zum Einzeltropfenkollektiv besitzt der Tropfen 9 zunächst eine gewisse Anfangsgeschwindigkeit am Zerfallsort. Während des freien Falls wird der Tropfen 9 beschleunigt, solange die treibende Kraft (Gewichtskraft minus Auftriebskraft) größer als die stetig sich erhöhende Widerstandskraft (Strömungskraft) ist. Daraus resultiert eine Fallgeschwindigkeit als Funktion der Zeit und Ortes bis bei gegebenem Kräftegleichgewicht zwischen den treibenden und den rückhaltenden Kräften eine stationäre Fallgeschwindigkeit $u_{T, stationär}$ erreicht wird. Es gilt bis zur Erreichung einer gleichförmigen Bewegung, dass die Geschwindigkeit des Tropfens 9 $u_T(t) < u_{T,stationär}$. Unter $u_T$(t = Zeit, Zeitintervall) wird die zeitabhängige Fallgeschwindigkeit des Tropfens 9 verstanden.

Die voneinander getrennten Tropfen des fließfähigen Ausgangsstoffes 9 werden in eine Erstarrungsflüssigkeit 11 überführt und müssen dabei eine Phasengrenze überwinden. Dabei kann aufgrund der Oberflächenspannung der Erstarrungsflüssigkeit 11 eine hohe Eintrittsbarriere und somit eine Schädigung der Tropfenform bewirkt werden. Es ist nun darauf zu achten, dass die aus der Oberflächenspannung resultierenden Kräfte möglichst minimiert werden und vielmehr das Eindringen des Tropfens des fließfähigen Ausgangsstoffes 9 in die Erstarrungsflüssigkeit 11 erleichtert wird. Dies bedeutet, dass die Oberflächenspannung der Erstarrungsflüssigkeit $\sigma_{Erstarrungsflüssigkeit}$ kleiner als 50 mN/m, insbesondere kleiner als 30 mN/m sein sollte und hierdurch der Übergang der Tropfen 9 rascher bewerkstelligt werden kann. Insbesondere bei stabilisierenden Erstarrungsflüssigkeiten, die eine entgegen gesetzte Polarität gegenüber dem fließfähigen Ausgangsstoff 2 besitzen (unpolar bei polarem fließfähige Ausgangsstoff 2, polar bei unpolarem fließfähigen Ausgangsstoff 2), wird eine hohe Grenzflächenspannung ausgebildet und die sphärische Tropfenform stabilisiert. Tropfen 9 und somit Feststoffpartikel 10 mit hoher Sphärizität werden bei einer Grenzflächenspannung zwischen dem Material der Tropfen 9 und der Erstarrungsflüssigkeit 11 zwischen 25 und 50 mN/m, insbesondere zwischen 30 und 50 mN/m und ganz besonders zwischen 35 und 50 mN/m erhalten.

Die Reduktion der Oberflächenspannung der Erstarrungsflüssigkeit 11 kann insbesondere bei polaren Erstarrungsflüssigkeiten 11 vorteilhaft durch den Zusatz von oberflächenaktiven bzw. oberflächenemiedrigenden Substanzen (z.B. Tenside) erreicht werden. Dem einschlägigen Fachmann sind vielfältige Möglichkeiten bekannt. Beispielhaft können die chemischen Funktionsklassen der Alky-/Arylsulfate, -sulfonate, -phosphate, -fluorate, -ethoxylate, Ether, Oxazolidine, Pyridinate, Sucinnate angeführt werden.

Das Maß einer eventuellen Schädigung der Tropfenform 9 an der Einbringungsstelle wird neben der Oberflächenspannung der Erstarrungsflüssigkeit 11 auch maßgeblich durch die kinetischer Energie der Tropfen 9, die zu einem gewissen Anteil beim Auftreffen in Verformungs- bzw. Deformationsarbeit umgewandelt wird, und dem Auftreffwinkel der Tropfen 9 auf die Oberfläche der Erstarrungsflüssigkeit 11 bestimmt. Es ist nun darauf zu achten, dass der Anteil der kinetischen Energie, der als Deformationsarbeit am Tropfen 9 umgewandelt wird, minimiert und optimiert wird. Hierzu ist die vektorell gerichtete Relativgeschwindigkeit $u_{relativ}$ zwischen dem Tropfen 9 und der Erstarrungsflüssigkeit 11 zu reduzieren und zu optimieren, vorteilhaft durch:

➢ die Reduktion der Fallhöhe bzw. der Fallzeit, so dass die zeitabhängige Fallgeschwindigkeit des Tropfens 9 $u_T$ (t) reduziert wird - dies bedeutet praktisch das Einbringen der Tropfen 9 unmittelbar bzw. kurz nach deren vollständiger Trennung zum Einzeltropfenkollektiv, insbesondere bei thermisch instabilen fließfähigen Ausgangsstoffen 2.
➢ die Veränderung des Auftreffwinkels.
➢ die Reduktion der Relativgeschwindigkeit $u_{relativ}$ zwischen dem Tropfen 9 und der Erstarrungsflüssigkeit 11.
➢ oder einer Kombination der oben angeführten Maßnahmen.

Zur Erzielung einer möglichst hohen Sphärizität müssen Schädigungen der existenten Feststoffpartikelform durch frei werdende Verformungsarbeit am Tropfen 9 beim Auftreffen auf die Oberfläche der Erstarrungsflüssigkeit 11 möglichst verhindert werden. Dies kann vorteilhaft dadurch erreicht werden, indem die Tropfen 9 unter einem spitzen Winkel $\alpha$ in die Erstarrungsflüssigkeit 11, insbesondere strömende Erstanungsflüssigkeit 11 eingebracht werden, das heißt $\alpha \leq 90°$, wobei der Winkel $\alpha$ als Winkel zwischen der Tangente an die Bewegungsbahnen 50 der Tropfen 9 und der Tangente an die Oberfläche der Erstarrungsflüssigkeit 11, jeweils angelegt an der Einbringungsstelle in die Erstarrungsflüssigkeit 11, insbesondere in eine strömende Erstarrungsflüssigkeit, definiert ist. Dieser Winkel ist in Fig. 2, 5, 6, 7, 10, 12, 13 und 14 in unterschiedlichen Ansichten und Ausführungsformen dargestellt.

Analoge Winkel können sich auch ergeben, wenn die Eintropfung in eine ruhende Erstarrungsflüssigkeit erfolgt und der Massenproportionierer 44 bewegt wird (siehe Fig. 15 und 16) oder die Bewegungsbahn der Tropfen 9 durch Neigung des Masseproportionieres 7 oder einer Kombination mit einer ruhenden und bewegten Erstarrungsflüssigkeit 11 eingestellt wird (siehe Fig. 7).

Weitere vorteilhaft anzuwendende Maßnahmen - zwecks Vermeidung einer Schädigung des Tropfens des fließfähigen Ausgangsstoffes 9 - beim Übertritt in die Erstarrungsflüssigkeit 11 finden sich in der Reduktion der vektoriell gerichteten und somit richtungsabhängigen bzw. wirksamen Relativgeschwindigkeit $u_{relativ}$ zwischen dem Tropfen 9 und der Erstarrungsflüssigkeit 11. Wie in z.B. Fig. 7 dargestellt, könnte durch Anpassung der Geschwindigkeit der Erstarrungsflüssigkeit 11 und der Fallgeschwindigkeit des Tropfens 9 an der Eintropfstelle die Relativgeschwindigkeit $u_{relativ}$ grundsätzlich 0 m/s eingestellt werden. Damit wirken in diesem Grenzfall auf den eintauchenden Tropfen 9 keine Kräfte auf Grund der Bewegung.

Dieser idealisierte Fall ist zwar für die Vermeidung einer Schädigung der Tropfen 9 vorteilhaft, doch ist häufig vorteilhaft, zumindest eine gewisse Relativgeschwindigkeit, in der Erstarrungsflüssigkeit 11 aufrecht zu erhalten.

Die Aufrechterhaltung einer häufig vorteilhaften, aber besonders vorteilhaft minimierten Relativgeschwindigkeit $u_{relativ}$ zwischen dem Tropfen 9 und der Erstarrungsflüssigkeit 11 an der Einbringstelle begründet sich auch in der rasch zu erfolgenden Überwindung der Phasengrenze. Besteht ein zu geringer Dichteunterschied zwischen den Tropfen 9 des fließfähigen Ausgangsstoffes und der Erstarrungflüssigkeit 11, ist es vorteilhaft die noch existente Überschuss-Geschwindigkeitsenergie für die Überwindung der Phasengrenze zu nutzen, da ansonsten die Tropfen 9 zum Aufschwimmen neigen, insbesondere bei strömenden und ganz besonders Erstarrungsflüssigkeiten, die unter einem spitzen Winkel geführt werden. In diesem Fall wird vorteilhaft ein größerer spitzer Winkel $\alpha$ eingestellt. Gerade bei der Zertropfung von Suspensionen eines keramischen Werkstoffes auf Basis $CeO_2/ZrO_2$ ist ein spitzer Winkel $\alpha > 15°$, insbesondere $> 45°$, insbesondere $> 60°$ und ganz besonders $> 70°$ einzustellen.

Entsprechend der oben beschriebenen Maßnahmen, die auf einen möglichst schädigungsarmen und quasi zerstörungsfreien Übergang der Tropfen 9 in die Erstarrungsflüssigkeit 11 abzielen, kann vorteilhaft sowohl die Härtung als auch der reformierende und/oder stabilisierende Schritt in der Erstarrungsflüssigkeit 11 - z.B. durch eine kühlende (härtende) und/oder reformierende und/oder stabilisierende Flüssigkeit bei der Herstellung von sphärischen Feststoffpartikel bewerkstelligt werden. In diesem Fall nutzt man das physikalische Prinzip der Paarung von entgegen gesetzten Polaritäten, In diesem Fall bildet sich die kleinste äußere Oberfläche eines geometrischen Körpers aus, dies ist eine Kugel. Es ist besonders vorteilhaft darauf zu achten, dass nach dem Abtauchen des noch fließfähigen Tropfens 9, dieser für die Formbildung noch ausreichende Beweglichkeit bzw. Fließfähigkeit zum Ausgleichen von Schädigungen besitzt.

Neben der Verbesserung der Sphärizität (Reformation) erfolgt in der Erstarrungsflüssigkeit 11 insbesondere die Härtung bzw. die Verfestigung zu den sphärischen Feststoffpartikeln 10 mit engen Korngrößen- Dichte- und Masseverteilungen. Die nachfolgend angeführten vorteilhaften Maßnahmen lassen sich insbesondere mit strömenden Erstarrungsflüssigkeiten 11 realisieren. Eine in dieser Phase (Partikel 10 noch nicht ausgehärtet) unerwünschte Koaleszenz (darunter wird die Koagulation von noch nicht gehärteten Partikel 10 verstanden) oder eine Aggregation (darunter wird das Verbinden von Einzelpartikel zu Partikelaggregaten verstanden), kann vorteilhaft durch eine kontinuierlich geführte Erstarrungsflüssigkeit 11 verhindert werden, die einen ausreichend schnellen Abtransport der erstarrenden Tropfen 10 und folgend einen ausreichenden Abstand der Einzeltropfen oder der späteren Einzelpartikel 10 zueinander garantiert.

Es ist weitergehend verständlich, dass bei einer noch ausreichend gegebenen Fließfähigkeit des abgetauchten und sphärischen Partikels 10 in der Erstarrungsflüssigkeit 11 Strömungskräfte eine Schädigung der Oberfläche und/oder der Form bewirken. Besonders vorteilhaft ist es, die Relativgeschwindigkeit zwischen dem absinkenden und/oder reformierenden sphärischen Partikel 10 und der Erstarrungsflüssigkeit 11 zu minimieren, das heißt der Partikel 10 sinkt im Grenzfall bei einer senkrecht ausgebildeten Bewegungsbahn 50 in der Erstarrungsflüssigkeit 11 mit einer konstanten Geschwindigkeit entsprechend dem Stokeschen Gesetz in einem ruhenden Medium aufgrund des Dichteunterschiedes. Darunter wird die Geschwindigkeit des umströmten Partikels 10 durch die Erstarrungsflüssigkeit 11 verstanden.

Häufig ist es vorteilhaft, aufgrund der Gewährleistung eines rasch zu erfolgenden Stoff- und Wärmeaustausches entsprechend hohe vektoriell definierte Relativgeschwindigkeit $u_{relativ}$ zwischen dem Partikel 10 und der Erstarrungsflüssigkeit 11 optimiert einzustellen. In Verbindung mit den beschleunigenden und verzögernden Effekten des erstarrenden Tropfen oder des Partikels 10 an der Einbringungsstelle bzw. nach dessen vollständiger Abtauchung, können die optimierten Strömungsverhältnisse durch die dimensionslosen Kennzahlen Reynolds [Re] und Froude [Fr] beschrieben werden.

Es ist besonders vorteilhaft, wenn die strömende Erstarrungsflüssigkeit 11 relativ zur Bewegungsgeschwindigkeit des Tropfens/Partikels an der Einbringungsstelle laminar geführt wird, das heißt eine Reynoldszahl [Re] von weniger als 2.320 aufweist und ganz besonders vorteilhaft laminare Strömungsverhältnisse des umströmten Partikels 10 im Bereich Re von 0,5 bis 500 und Froude Fr von 0,1 bis 10, besonders weniger als 5 und ganz besonders von weniger als 2 optimiert eingestellt sind. Die Werte zur Beschreibung der Strömungsverhältnisse werden auf den abgetauchten und umströmten Partikel kurz nach der Einbringungsstelle bezogen.

Die optimierte Einstellung der laminaren Strömungsverhältnisse der Erstarrungsflüssigkeit, insbesondere kurz vor der Einbringungsstelle, kann durch longitudinale oder rotierende Strömungen verwirklicht werden, insbesondere durch ausgeprägte und/oder besonders vorteilhaft voll entwickelte Strömungen der longitudinalen und rotierenden Strömungsarten. Unter ausgeprägten und voll entwickelten Strömungen werden definierte Strömungen (z.B. Trombe, Drall) und/oder insbesondere speziell geführte Strömungen verstanden (Wandbegrenzungen, Rinnenströmung usw.). Diese Strömungen besitzen besonders die Vorteile, dass Wirbelbildungen und/oder Wandberührungen verringert werden können. Die vorteilhaften Ausführungsformen werden in Zusammenhang mit den Figuren beschrieben :

Es ist weitergehend vorteilhaft, wenn aufgrund des Auftretens von Kräftepaaren zwischen dem Tropfen 9 und der unter einem bestimmten Winkel geführten Erstarrungsflüssigkeit 11, ein Drehimpuls induziert wird (Fig. 8, 9), welches zu einer erwünschten Drehbewegung bzw. zu einer Rotation des Tropfens 9 führt: Diese induzierte Drehbewegung stabilisiert den Tropfen 9 weitergehend bzw. unterstützt folgend auch die Reformation zu einem sphärischen Feststoffartikel 10. Dieser Einfluss kann vorteilhaft durch den Neigungswinkel und die Relativgeschwindigkeiten gesteuert werden und/oder durch Aufprägen von Geschwindigkeitsfeldem in zwei Achsen, z.B. durch eine zusätzliche Querkomponente - z.B. durch eine zusätzliche Tangentialströmung in einem Trichter mit Überlaufkante neben der Hauptströmungsrichtung (Horizontalströmung bzw. Vertikalströmung in einem Trichter mit Überlaufkante) der Flüssigkeitsbewegungvorteilhaft genutzt werden.

Erfolgt die Härtung durch Kühlung bietet eine Erstarrungsflüssigkeit 11 und insbesondere eine strömende Erstarrungsflüssigkeit im Vergleich zu einer Kühlung in Gasphase, aufgrund der höheren Wärmekapazität, Dichte und Wärmeleitfähigkeit der Erstarrungsflüssigkeit 11 wesentliche Vorteile. Dabei werden sowohl der Wärmeaustausch als auch insbesondere bei chemisch härtenden Systemen der Stoffaustausch durch die eingestellten Strömungsverhältnisse wesentlich gegenüber Gasphasen und/oder ruhenden Erstarrungsflüssigkeiten erhöht. Vorteilhaft erfolgt eine weitergehende Erhöhung sowohl der Wärme- als auch der Stoffübergangskoeffizienten. Überdies sind vorteilhaft stationäre Anfangsbedingungen garantiert - z.B. Temperatur, Konzentration an der Eintrittsstelle des Tropfens 9 in die strömende Erstarrungsflüssigkeit 11 - und stellen insofern vorteilhaft optimal einzustellende Parameter dar.

Bei Suspensionen auf Basis eines keramischen Werkstoffs und eines Bindmittels kann durch die Variation der Temperatur der Erstarrungsflüssigkeit die Formations- und/oder chemischen Härtungszeiten bewusst gesteuert werden.

Konditionierung der Feststoffpartikel

Die Verwendung einer gering oder nicht benetzenden Erstarrungsflüssigkeit 11 kann vorteilhaft auch bei der Lagerung der sphärischen Feststoffpartikel 10 genutzt werden. Bevorzugt ist es, wenn die Partikel 10 mit Amino- und/ oder Oxitriazinen und/oder Kohlenwasserstoffen konditioniert vorliegen.

Die Konditionierung führt zu einer besseren Rieselfreudigkeit der Feststoffpartikel und verhindert ein Zusammenbacken.

Die Konditionierungsmittel können auch nachträglich durch Aufsprühen und/oder Granulieren auf das fertige Feststoffpartikel 10 aufgebracht werden. Besonders bevorzugt ist es, wenn ein bei der Herstellung des Feststoffpartikels 10 verwendetes Fluid (Erstarrungsflüssigkeit 11) gleichzeitig als Konditionierungsmittel dient. Auf diese Weise können die Kugelgenerierung und die Konditionierung in einem Verfahrensschritt erfolgen.

Das Verfahren zur Erzielung von Feststoffpartikeln mit einer hohen Sphärizität, insbesondere sphärischen Kornform und engen Komgrößen-, Masse- und Dichteverteilungen weist zusammenfassend insbesondere folgende Aspekte auf:

1. Einstellung und Konstanthaltung eines Massestromes eines fließfähigen Ausgangsstoffes 2 zur Erzielung einer eng verteilten monomodalen Masseverteilung der zu erzeugenden Tropfen 9 oder Feststoffpartikel 10.

2. Masseproportionierung 7, 8 bzw. Tropfenerzeugung 9 entsprechend der Gesetze des laminaren Strahlzerfalls ohne oder mit Resonanzanregung, insbesondere in den über dimensionslose Kennzahlen beschreibbaren Strömungsregimen des Abtropfens und Zertropfens (laminarer Strahlzerfall).

3. Gewährleistung eines zerstörungsarmen, insbesondere zerstörungsfreien, Übergangs der erzeugten Tropfen 9 in eine Flüssigphase einer Erstarrungsflüssigkeit 11 (Überwindung einer Phasengrenze).

4. Gewährleistung eines zerstörungsarmen, insbesondere zerstörungsfreien, und rasch erfolgenden Abtransports der Partikel durch die Erstarrungsflüssigkeit 11 zwecks Verhinderung von Koaleszenz und/oder Aggregation der Tropfen des fließfähigen Ausgangsstoffes unter der Prämisse der Verhinderung von Schädigungen durch die jeweils vorherrschenden Strömungskräfte.

5. Reformation und/oder Stabilisierung der Tropfen des fließfähigen Ausgangsstoffes zu sphärischen Feststoffpartikeln 10 durch die Erstarrungsflüssigkeit 11 unter Bedachtnahme einer mehr oder weniger rasch erfolgenden Härtung zu den sphärischen Feststoffpartikeln 10.

6. Gewährleistung einer ausreichenden Härtung innerhalb der Erstarrungsflüssigkeit 11 zwecks Manipulation der sphärischen Feststoffpartikeln 10.

7. Konditionierung der sphärischen Feststoffpartikel.

Die Eintropfung für das oben beschriebene Verfahren wird am Beispiel der Fig. 3, 6 und 13 nachfolgend erklärt. Der Masseproportionierer 7, 8 bewirkt die Zerteilung des Strahls in masseeng verteilte Tropfen 9 - entsprechend der oben angeführten Beschreibung zu Fig. 4.

Die schädigungs- und zerstörungsfreie Überführung der Tropfen 9 in die Erstarrungsflüssigkeit 11 z.B. durch die Maßnahmen der Oberflächenspannung ($\sigma_{Erstarrungsflüssigkeit}$ < $\sigma_{Tropfen}$), der Einstellung eines Winkels a sowie die Reformation/Stabilisierung (Grenzflächenspannung, Polaritätsunterschied), Härtung (Kühlmittel) und / oder der Abtransport (strömend) der sphärischen Feststoffpartikel 10 ist im Detail in Fig. 5 und in einer besonderen Ausführungsform in Fig. 10 dargestellt.

Nach der Überführung der Tropfen 9 in die Erstarrungsflüssigkeit 11 reformieren und härten die Tropfen 9 zu sphärischen Feststoffpartikeln 10. Die Tropfen 9 folgen hier im Wesentlichen einer senkrechten Bewegungsbahn 50.

In Fig. 2 ist ferner dargestellt, dass die im Eintropfapparat bzw. im Rinnenkanal formstabilisierten und gehärteten und sphärischen Feststoffpartikel 10 in einen Vorratsbehälter 13 für die Erstarrungsflüssigkeit 11 gelangen. Mittels einer mechanischen Trenneinheit 12 - z.B. ein Siebkorb- werden die gehärteten und sphärischen Feststoffpartikel 10 von der Erstarrungsflüssigkeit 11 separiert.

Weitere vorteilhafte Ausführungsformen werden in Zusammenhang mit den Figuren beschrieben,

Eine ausgeprägte insbesondere voll entwickelte Strömung der Erstarrungsflüssigkeit 11 wird bevorzugt durch eine voll entwickelte Gerinneströmung, insbesondere in Form eines Rinnenkanals, definiert. Eine voll entwickelte Strömung, insbesondere in einem Rinnenkanal des Eintropfapparats ist in Fig. 5 dargestellt. Die Erzeugung des vorteilhaft nutzbaren Winkels $\alpha$ wird mit einem strömungsmechanisch speziell geformte Überlaufwehr 31 bewirkt, das eine sehr sanfte Umlenkung der Erstarrungsflüssigkeit 11 (Kühlflüssigkeit) bewirkt, wobei die Kontur des Überlaufwehres 31 durch die Kühlflüssigkeit an deren Oberfläche übernommen bzw. abgebildet wird und folgend der spitze Winkel $\alpha$ zwischen der Tangente an die Bewegungsbahn 50 der Tropfen 9 und der Tangente an die Oberfläche der strömenden Erstarrungsflüssigkeit, jeweils angelegt an der Einbringungsstelle in die strömende Erstarrungsflüssigkeit 11, erzeugt wird.

In speziellen Ausführungsformen des Rinnenkanals bzw. der voll entwickelten Gerinneströmung wird anstelle des speziell geformten Überlaufwehrs 31 ein strömungsmechanisch speziell geformter Strömungsstörkörper 31 (siehe Fig. 12) oder besonders vorteilhaft ein verstellbarer Strömungsstörkörper 31 in Form eines Tragflügels (siehe Fig. 13) eingesetzt. Beide Ausführungsformen bewirken wiederum die Aus- bzw. Abbildung eines spitzen Winkels a zwischen der Tangenten an die Bewegungsbahnen 50 der Tropfen 9 und der Tangente an die Oberfläche der strömenden Erstarrungsflüssigkeit, jeweils angelegt an der Einbringungsstelle in die strömende Erstarrungsflüssigkeit.

Der Tragflügelströmungsstörkörper (Fig. 13) besitzt die Vorteile einerseits in der raschen Anpassung bzw. Veränderung des sich abbildenden Winkels und andererseits in der Einstellung einer Unterströmung, so dass besonders vorteilhaft ein rascher Abtransport der sphärischen Partikel 10 vom Eintropfbereich bewirkt werden kann.

Auch das Eintropfen in einen Trichter mit überlaufender Erstarrungsflüssigkeit hat einen ähnlichen Effekt (Fig. 10, 11). In den Trichter können Leitbleche eingesetzt werden, so dass wiederum eine voll ausgebildete Gerinneströmung vorteilhaft realisiert werden kann. Die Erstarrungsflüssigkeit 11 wird entweder vertikal und entgegen der Gravitationsrichtung über ein nach unten gekrümmtes Rohr zugeführt oder/und kann durch tangential angeordnete Zuführrohrleitungen in eine Drallbewegung versetzt werden. Die erste Rohranordnung garantiert die vertikale Förderung der Erstarrungsflüssigkeit, so dass eine sehr beruhigte und glatte Oberfläche eingestellt werden kann. Die zweite Rohranordnung bewirkt die Drallbewegung unter beruhigten Strömungsverhältnissen. Die Strömungen sind voll entwickelt. Eine weitere Beruhigung der Strömung wird durch das Aufweiten der kreisrunden Trichterkonstruktion vom Boden in Richtung Flüssigkeitsniveau bewirkt - entspricht einer Art Diffusor.

Mit Hilfe eines speziell geformten Überlaufwehres 31 erfolgt die störungsfreie Überführung der Erstarrungs-

flüssigkeit 11 in einen Trichterbereich. Das speziell geformte Überlaufwehr 31 wird an der Außenseite des Trichters tangential von dessen Neigung in eine sanfte kreissegmentartige Rundung überführt, an diese schließt eine Art parabolisch geformte Rundung an, deren Schenkel in Richtung des innen liegenden Trichters sehr flach verläuft (siehe Fig. 18). Dadurch kann die Flüssigkeit über einen längeren Zeitraum auf annähernd gleichem Niveau gehalten werden. Der Übergang von dem parabolischen Segment zur innen liegenden Trichterwand verläuft wiederum tangential gerichtet über eine Art stärker gekrümmtes Kreissegment. Alle gekrümmten Segmente bilden in sich eine Einheit und aufgrund der tangentialen Übergänge zu den Trichterwandungen ebenfalls eine nach außen erscheinende geschlossene Einheit. Ein weiterer Vorteil dieser Formgebung findet sich in der Bereitstellung einer ausreichend hohen Filmdicke der Erstarrungsflüssigkeit 11 im Leitrinnenkanal. Dadurch kann vorteilhaft eine vorzeitige Berührung des noch nicht ausreichend gehärteten Partikels 10 mit der Wand vermieden werden. Im konkreten Anwendungsfall werden Flüssigkeitshöhen von 20-40 mm vorteilhaft, gemessen als Abstand zwischen der Tangente der horizontal orientierten Überlaufkante zum Flüssigkeitsniveau, eingestellt.

Die Formation als auch der Abtransport der sphärischen Feststoffpartikel 10 erfolgt vorteilhaft durch die jeweils vorherrschende Strömungsgeschwindigkeit der Kühlflüssigkeit (Erstarrungsflüssigkeit 11). In einem konkreten Anwendungsfall beträgt diese an der horizontalen Überlaufkante etwa 0,2 bis 0,8 m/s, wobei sich dieser Wert nur unwesentlich durch die spezielle Form des Überlaufwehres als Funktion der Fallhöhe verändert.

Die geometrische Ausbildung des speziellen Überlaufwehres 11 erfolgt strömungsmechanisch. Im konkreten Anwendungsfall, zeigen sich laminare Strömungsverhältnisse relativ zum Feststoffpartikel 10 bzw. Re-Zahlen von weniger als 2.320, insbesondere zwischen 0,5 und 500, sowie Froude-Zahlen von weniger als 10, besonders von weniger als 5 und ganz besonders von weniger als 2.

In einer speziellen Ausführungsform des Rinnenkanaltrichters (Fig. 10, 11) werden Leitbleche, insbesondere verjüngte Leitbleche zur strömungsmechanischen Führung der Strömung bzw. zur Ausbildung einer voll entwickelten Gerinneströmung in den Trichter eingebracht. Die Leitbleche sind nach unten verjüngt, so dass auch entlang der geneigten Trichterwand eine ausreichende Flüssigkeitshöhe ausgebildet bleibt und folgend eine Wandberührung der sphärischen Feststoffpartikel 10 verhindert werden kann. Die Leitbleche können insbesondere auch gekrümmt ausgeführt werden, so dass der Vorteil der Drallbewegung bzw. die 2-dimensionalen Strömungsfelder genutzt werden kann.

Aufgrund der Kreissymmetrie des Trichters (siehe Fig. 11) mit oder ohne Leitbleche, kann vorteilhaft eine kreissymmetrische Vertropfungseinrichtung mit mehreren Düsen, angeordnet werden. Aufgrund der rasch erfolgenden Prozesse ist eine Modulbauweise zwecks Kapazitätserhöhungen vorteilhaft erreichbar, in dem mehrere Trichter und Vertropfungseinrichten in einem Fallrohr angeordnet werden.

In einer weiteren Ausführungsform (Fig. 5) wird anstelle des Trichters eine Rinne eingesetzt. Die Zuführung des Kühlmediums erfolgt analog zu der zuvor angeführten Beschreibung über einen Kasten, der die vertikal orientierten Rohrzuführungen aufweist, so dass wiederum eine glatte und strömungsmechanisch optimale Zuführströmung resultiert. Die Strömung wird entlang von Wänden gerichtet und in Richtung eines speziell geformten Störkörpers, der jenem des Überlaufwehres der Fig. 10 entspricht, gelenkt. Die Strömung ist wiederum voll entwickelt. In diesem Fall wird über die Länge der Gerinneströmung die für die Formation und Härtung notwendige Verweilzeit in Verbindung mit der Strömungsgeschwindigkeit definiert. In diesem Fall können durch die Breite der Rinne auch entsprechend höhere Flüssigkeitshöhen vorteilhaft eingestellt werden.

In einer weiteren Ausführung (Fig. 14) werden die Maßnahmen zur Erzeugung eines sphärischen Feststoffpartikels 10 durch die Ausbildung einer ausgeprägten Rotationsströmung, insbesondere durch die Ausbildung einer Trombenform 61 in einem Rührkessel 60 bewerkstelligt. Mit Hilfe eines am Boden angeordneten Rührorgans 63, dessen Drehzahl 64 zur Einstellung einer definierten Geschwindigkeit sowie der Abstand zur Flüssigkeitsoberfläche variiert werden können, wird eine sanfte Trombenform ausgebildet und folgend ein Winkel $\alpha$ zwischen der Tangente an die Bewegungsbahnen 50 der Tropfen 9 und der Tangente an die Oberfläche der strömenden Erstarrungsflüssigkeit 11, jeweils angelegt an der Einbringungsstelle in die strömende Erstarrungsflüssigkeit, erzeugt. Aufgrund der Drallbewegung und unter den Einflüssen der Zentrifugal- und Corioliskräfte zeigen die Partikel 10 eine schraubenförmige Bewegungsbahn, wodurch die Verweilzeit entsprechend vorteilhaft verlängert wird.

In einer weiteren bevorzugten Ausführungsform wird ein rotierender Behälter bzw. eine rotierende Erstarrungsflüssigkeit 11 zur Herstellung von Feststoffpartikeln 10 nach (Fig. 17) eingesetzt. Dabei wird im Außenbereich ein durch die Wände von 2 Zylindern (Ring) begrenzter kreisförmiger Rinnenkanal ausgebildet, so dass eine voll entwickelte Rotationsströmung erzeugt wird. In dieser speziellen Ausführungsform wird die Erstarrungsflüssigkeit 11 über eine Gleitringdichtung am Boden des Behälters 201 zugeführt. Die Erstarrungsflüssigkeit 11 wird über ein Steigrohr 202 in eine ringförmige Verteilvorrichtung 203/204 mit Eintrittsöffnungen, insbesondere Lochöffnungen 205 in den eigentlichen Eintropfbereich 206 gefördert. Die Eintrittsöffnungen 205 der Verteilervorrichtung sind knapp unterhalb Erstarrungsflüssigkeitsoberfläche, etwas unterhalb der eigentlichen Eintropfstelle angeordnet. Durch diesen Abstand wird eine eventuell störende Longitudinalbewegung der Erstarrungsflüssigkeit 11 auf die Feststoffpartikel 10 verhindert. Bei einer Bewegungsbahn 50 des Tropfens 9 senkrecht zur Oberfläche der Erstarrungsflüssigkeit ist a = 90°. Die getrennten Tropfen 9 des Masseproportionierers erfahren beim Phasenübergang, durch das Drehmoment der Anströmung eine vorteilhafte Spinbewegung und werden durch die Rotation des Behälters 211 bzw. der Erstarrungsflüssigkeit 11 in eine schraubenförmige Bewegung versetzt, wodurch die Verweilzeit entsprechend verlängert wird. Durch die spezielle Konstruktion des Eintropfbereichs bildet sich eine beruhigte Oberfläche der Erstarrungsflüssigkeit aus. Bei höheren Umfangsgeschwindigkeiten können alternativ auch bestimmte Neigungswinkel der nach außen durch die Zentrifugalkraft angehobenen bzw. schiefen Oberfläche der Erstarrungsflüssigkeit 11 realisiert werden, dies bedeutet einen Winkel von α< 90°. Sowohl die sphärischen Feststoffpartikel 10 als auch die Erstarrungsflüssigkeit 11 werden durch die Strömung in den Bodenbereich des rotierenden Behälters gezwungen. Im Bodenbereich erfolgt entweder durch einen konisch und sich erweiternden ausgebildeten Sammelbereich 209 die Trennung der Feststoffpartikel 10 durch Gravitation oder durch ein dort installiertes Siebgeflecht von der leicht erwärmten Erstartungsflüssigkeit 11. Die Erstarrungsflüssigkeit 11 steigt entgegen der Schwerkraft in den Ableit- bzw. Rückführbereich 207, der durch einen geodätisch etwas tiefer angeordneten innen liegenden Trichter gegenüber dem eigentlichen Niveau der Erstarrungsflüssigkeit 11 an der Eintropfstelle ausgebildet wird. Das Ausbringen der sphärischen Partikel 10 erfolgt durch diskontinuierliches Öffnen des Absperrorgans 210, wobei die sphärischen Partikel 10 zusammen mit einem geringen Teil der Erstarrungsflüssigkeit 11 aufgrund der Zentrifugalkräfte aus dem Behälter in einen außenliegenden Auffang- und Trennapparat geschleudert werden. Alle anderen Anlagenkomponenten, wie z.B. Masseproportionierer, Wärmetauscher gleichen den bisherigen Beschreibungen.

In einer weiteren besonders bevorzugten Ausführungsform (Fig. 18) der voll entwickelten und laminar geführten Rotationsströmung wird die Erstarrungsflüssigkeit 11 tangential 302, 303 in den ringförmig ausgebildeten Bereich (zwei Zylinder) eines stehenden Behälters zugeführt. Ein weiterer Unterschied zum zuvor angeführten rotierenden Behälter findet sich in der geschlossenen Bauweise des Apparates - der innen liegende Zylinder (kein Trichter zur Ableitung der Fluidphase) ist nach oben geschlossen. Die Effekte gleichen jenen des rotierenden Behälters mit der Ausbildung einer schraubenförmigen Bewegung 305 der Feststoffpartikel 10 und der vorteilhaften Verlängerung der Verweilzeit sowie der möglichen Einstellung einer geneigten Oberfläche der Erstarrungsflüssigkeit 11 bei entsprechend hohen Umfangsgeschwindigkeiten. Die Abtrennung der Feststoffpartikel von der Erstarrungsflüssigkeit erfolgt in gewohnter Weise mit einer bekannten Trennvorrichtung wie beispielsweise einem Zyklon 307 oder über ein Drahtgeflecht oder Sieb 12. Der Vorteil des Apparates findet sich in der quasi kontinuierlich gestaltbaren Ausschleusung der sphärischen Feststoffpartikel 10 über die Absperrarmatur 308, wobei durch das geschlossene System das Niveau 102 der Erstarrungsflüssigkeit 11 gehalten werden kann bzw. die Nachspeisung über einen Füllstandsmesser 16 bewirkt wird. Alle anderen Anlagenkomponenten, wie z.B. Masseproportionierer, Wärmetauscher gleichen den bisherigen Beschreibungen. In Fig. 19 ist eine Zertropfung eines fließfähigen Ausgangsstoffes, insbesondere einer Suspension auf Basis eines keramischen Werkstoffes und eines Bindemittels, in eine ruhende Erstarrungsflüssigkeit 11 dargestellt. Diese weist zwei miteinander wenig oder nicht mischbare Phasen bzw. Stoffe unterschiedlicher Polaritäten und/oder unterschiedlicher Dichten auf. Die getrennten Tropfen 9 des Masseproportionierers werden in diesem Fall in eine unpolare und leichte Phase der Erstarrungsflüssigkeit 11, die eine geringe Oberflächenspannung, insbesondere von weniger als 30 mN/m aufweist, eingeleitet. In dieser ersten Phase der Erstarrungsflüssigkeit erfolgt überwiegend die Reformation der noch fließfähigen Tropfen 9 zu sphärischen, noch fließfähigen Tropfen 9. Die Verfestigung bzw. Härtung erfolgt in der zweien, dichteren Phase der Erstarrungsflüssigkeit 11 zu den sphärischen Feststoffpartikeln 10. Dabei ist auf eine geringe Grenzflächenspannung zwischen der leichteren und dichteren Phase der Erstarrungsflüssigkeit insbesondere zu achten. Diese sollte vorteilhafterweise einen Wert von weniger als 10 mN/m aufweisen. Die gehärteten sphärischen Feststoffpartikel 10 werden in gewohnter Weiser über ein eine Trenneinrichtung, z.B. über ein Sieb oder Filter 12 von der schwereren Phase der Erstarrungsflüssigkeit abgetrennt und die separierte Erstarrungsflüssigkeit wiederum dem Apparat zugeführt. Alle anderen Anlagenkomponenten, wie z.B. Masseproportionierer, Wärmetauscher gleichen den bisherigen Beschreibungen.

Alternativ oder auch in Kombination dazu kann ein reines Abtropfverfahren verwendet werden, bei dem die Tropfen 9 nicht durch Zerteilung einer laminaren Strömung erzeugt werden.

In Fig. 15 ist schematisch ein einfache Vorrichtung dargestellt, die eine Lochplatte 40 aufweist. Diese Lochplatte 40 ist unterhalb eines Reservoirs 41 für den fließfähigen Ausgangsstoff, angeordnet. In der Lochplatte 40 ist eine Vielzahl von einzelnen Düsen 42 angeordnet, die im einfachsten Fall Bohrungen in der Lochplatte 40 darstellen. Alternativ können die Düsen auch eine von oben nach unten verjüngende, trichterartige Kontur aufweisen, so dass der fließfähige Ausgangsstoff gut durch die Düsen 42 geführt wird. Bei Anlegung einer Druckdifferenz über die Düsenplatte 40, tropfen einzelne Tropfen 9 aus den Düsen 42, wobei die Lochplatte 40 mit den Düsen 42 als Masseproportionierer fungiert.

Da der Strömungsvorgang hierbei nicht extern, z.B. durch Schwingungen angeregt wird, bilden sich die Tropfen 9 allein unter der Schwerkraft. Dies dauert in der Regel länger als eine hochfrequente Anregung der Zertropfungseinheiten. Allerdings weist die Ausführungsform den Vorteil auf, dass eine große Menge Düsen 42 auf einer Lochplatte 40 angeordnet werden kann.

Die Erstarrung der Tropfen 9 zu Feststoffpartikeln 10 kann dann in einer Weise erfolgen, wie sie in den anderen Ausführungsformen beschrieben wurde.

Bei einer weiteren Ausführungsform gemäß Fig. 16 wird die Strömungsgeschwindigkeit für eine Abtropfung durch eine Zentrifugalkraft generiert. Fig. 16 zeigt eine perspektivische Ansicht einer runden Lochplatte 40, an deren Umfang eine Wandung 43 angeordnet ist. Die Wandung 43 bildet zusammen mit der Lochplatte 40 das Reservoir 41.

Die Düsen 42 für den Durchtritt des fließfähigen Ausgangsstoffes sind am Umfang der Lochplatte 40 angeordnet. Durch eine Zuleitung 44 wird der fließfähige Ausgangstoff in das Reservoir gebracht, wobei die Zuleitung 44 bei der Förderung in Rotation versetzt wird. Dadurch erfährt der austretende fließfähige Ausgangsstoff eine Beschleunigung nach Außen in Richtung Wandung 43; der Ausgangsstoff wird gegen die Wandung 43 gedrückt. Durch Einstellung der Förderungsgeschwindigkeit, der Rotation und der Füllhöhe kann ein definierter Druck an den Düsen eingestellt werden. Die Düsen 42 fördern dann den fließfähigen Ausgangstoff von der Düsenplatte 40 weg.

Grundsätzlich kann diese Ausführungsform auch so ausgebildet sein, dass die Zuleitung 43 ruht und die Lochplatte 40 rotiert, In diesem Fall sind die Düsen 42 in der Wandung 43 angeordnet. Die Erfindung enthält die Erkenntnis, dass bei Einhaltung der oben genannten Verfahrensschritte verschiedenste Feststoffpartikel 10 mit hoher Sphärizität und enger Größenverteilung hergestellt werden können.

Die erfindungsgemäßen Feststoffpartikel aus einem keramischen Werkstoff sind gekennzeichnet durch

(a) eine Sphärizität von ≥ 0,930,

(b) einen Durchmesser zwischen 20 $\mu$m und 6.000$\mu$m, bei einer relativen Standardabweichung von ≤ 10%.

woher der keramische Werkstoff ein cerstabilisiertes Zirconoxid mit einem $CeO_2$-Gehalt von 10 bis 30 Massen -% ist.

Eine bevorzugte Ausführungsform der Feststoffpartikel aus einem keramischen Werkstoff ist durch eine Sphärizität von größer ≥ 0,960, insbesondere von ≥ 0,990, gekennzeichnet. Weitere bevorzugte Ausführungsformen der Feststoffpartikeln aus einem keramischen Werkstoff sind durch einen Durchmesser zwischen 100 $\mu$m und 2.500 $\mu$m, bei einer relativen Standardabweichung von ≤ 5%, bevorzugt ≤ 4%, insbesondere ≤ 1%, und darüber hinaus durch einen Durchmesser zwischen 300 $\mu$m und 2.000 $\mu$m, bei einer relativen Standardabweichung von ≤ 3,5%, gekennzeichnet.

Als Mahlkörper in Mühlen, insbesondere Hochleistungsmühlen, lassen sich beispielsweise diese keramischen Feststoffpartikel verwenden, Ferner sind diese Feststoffpartikel durch eine Kornrohdichte (nach Sinterung) im Bereich zwischen 6,100 und 6,250 g/cm$^3$ gekennzeichnet.

Die Sphärizität wird aus dem Minimum- und Maximum-Feret-Durchmesser errechnet, welche in der Norm DIN 66141 definiert sind und nach der ISO-Norm CD 13322-2 bestimmt werden.

Die Sphärizität ist ein Maß für die Exaktheit der Rollbewegung eines Feststoffpartikels 10, insbesondere während der Förderung in einem Dosierapparat. Eine hohe Sphärizität, idealerweise eine Kugel (Sphärizität = 1), führt zu einer Senkung des Rollwiderstandes und verhindert eine Taumelbewegung durch nicht sphärische Oberflächenabschnitte wie beispielsweise Flachstellen, Dellen oder Erhebungen. Damit wird die Dosierbarkeit erleichtert.

Als Kornrohdichte, insbesondere die mittlere Kornrohdichte wird gemäß der E-Norm 993-17 DIN-EN aus dem Jahr 1998 das Verhältnis der Masse einer Menge der Körner (d. des Werkstoffs) zum Gesamtvolumen der Körner einschließlich des Volumens von geschlossenen Poren in den Körnern verstanden.

Gemäß der Norm wird die Kornrohdichte nach dem Verfahren der Quecksilberverdrängung unter Vakuumbedingungen gemessen. Dabei werden unter Anwendung eines bestimmten Druckes kreisrunde und spaltförmige, insbesondere offene Poren mit definiertem Durchmesser mit Quecksilber gefüllt und so das Volumen des Werkstoffes bestimmt. Über die Masse des Werkstoffes (d.h. der Körner) wird auf diese Weise die Kornrohdichte, insbesondere die mittlere Kornrohdichte errechnet.

Der Minimum-Feret-Durchmesser und der Maximum-Feret-Durchmesser sind definiert in der Norm DIN 66141 und werden bestimmt nach der ISO-Norm CD 13322-2, welche die Komgrößenbestimmung von Stoffen durch die dynamische Bildanalyse behandelt. Dabei werden digitale Momentaufnahmen der Partikel, die beispielsweise über eine Förderrinne dosiert werden und herabfallen, aufgenommen. Die digitalen Momentaufnahmen geben die projizierten Flächen der einzelnen Partikel in den verschiedenen Positionen der Bewegung wieder. Aus den digitalen Momentaufnahmen werden Messdaten über Korndurchmesser und Kornform für jedes einzeln registrierte Partikel berechnet und über die Gesamtzahl der pro Probe erfassten Partikel statistische Auswertungen durchgeführt.

Zur Bestimmung der Korndurchmesser und Kornform werden die Feret-Durchmesser herangezogen. Der Feret-Durchmesser ist der Abstand zweier Tangenten, die senkrecht zur Messrichtung an das Korn angelegt werden. Der Minimum-Feret-Durchmesser ist somit der kürzeste Durchmesser eines Korns, der Maximum-Feret-Durchmesser der längste Durchmesser eines Korns.

Die Sphärizität der Partikel wurde gemessen mit dem Gerät Camsizer 187 (Retsch Technology, Softwareversion 3.30y8, Einstellungsparameter: Verwendung einer CCD-Zoomkamera, Flächenlichtquelle, 15mm Rinne, Leitblech, 1% Partikeldichte, Bildrate 1:1, Messung in 64 Richtungen) entsprechend der ISO-Norm CD 13322-2 und ausgewertet nach DIN 66141. Die Messung beruht auf dem Prinzip der dynamischen Bildanalyse, die Sphärizität SPHT ist definiert als

$$SPHT = \frac{4\pi A}{U^2}$$

mit A = projizierte Fläche des Partikels und U = Umfang des Partikels

Für die projizierte Bildfläche eines Kreises, d.h. eines sphärischen Partikels gilt SPHT = 1, bei abweichenden Partikelformen SPHT < 1. Die Sphärizität ist ein Maß, das die Abrollbarkeit der Partikel bei der Förderung charakterisiert. Eine gute Abrollbarkeit der Harnstoffpartikel 10 führt zu einer Senkung des Förderungswiderstandes und minimiert die Neigung der Hamstoffpartikel 10 sich zu verhaken. Damit wird die Dosierbarkeit erleichtert.

**Beispiele:**

Mit den nachfolgenden Beispielen wird die Erfindung noch näher erläutert.

Beispiel 1;

Entsprechend der oben beschriebenen Versuchsanordnung wurden als Feststoffpartikel sphärische Feststoff-partikel auf Basis einer Keramik (10) mit einem mittleren Durchmesser dso von etwa 0,43 mm mit dem Rinnenkanaltrichter (Fig. 5) hergestellt.

Eine wässerige Suspension 2 der Oxide des Stoffsystems $CeO_2/ZrO_2$ mit 16,3 Masse-% $CeO_2$, bezogen auf die Einsatzoxide, wurde nach der Nasszerkleinerung mit 0,45 Masse-% des keramischen Bindemittels Ammoniumalginat versetzt. Die wässerige Suspension wurde anschließend mit dem Dispergierorgan der Firma IKA, Type: Ultra Turax D50, dispergiert bzw. das keramische Bindmittel in der wässerigen Suspension der Oxide homogenisiert. Die dispergierte Suspension wies eine Restfeuchte von 48,5 Masse-%, eine dynamische Viskosität von 3,6 dPas und eine Oberflächenspannung von 43,5 mN/m auf.

Zur Herstellung von sphärischen keramischen Partikeln in einem Durchmesserbereich zwischen 0,36 bis 0,55 mm (nach der Sinterung) wurde 1 $dm^3$ der oben angeführten Fertigsuspension in einem Laborrührwerksbehälter von 2 $dm^3$ gefüllt. Die Fertigsuspension wurde kontinuierlich mittels eines langsam laufenden Ankerrührorgans 3 gerührt. Die Drehzahl des Rührorgans betrug 60 U/min.

Als härtende, stabilisierende und formgebende Erstarrungsflüssigkeit 11 wurde eine wässerige alkoholische Calciumchlorid-Lösung eingesetzt. Es wurde eine Erstarrungsflüssigkeit 11 aus zwei miteinander komplett mischbaren Stoffen unterschiedlicher Polarität hergestellt.

Die Konzentration der im Vergleich zum zertropfenden Medium (Fertigsuspension) weniger polaren Komponente Ethanol betrug 25 Masse-%. In der ethanolischen Lösung wurde 1 Masse-% $CaCl_2$ gelöst. In diesem Fall kann eine Oberflächenspannung von 42,5 mN/m der alkoholischen $CaCl_2$-Lösung gemessen werden. Diese ist kleiner als jene der Fertigsuspension mit 43,5 mN/m. Die Dichte der härtenden Lösung betrug 1.001 $kg/dm^3$.

Die Lösung wurde wie oben beschrieben vom Vorratsbehälter über eine Kreiselpumpe zum Masseproportionierer gefördert. Die Härtung erfolgt durch die zweiwertigen Calciumionen in Verbindung mit dem zugesetzten keramischen Bindemittel Ammoniumalginat.

Das Schwingsystem wurde aktiviert.

Bei gegebener Betriebsbereitschaft wurde das Schwingungssystem zur Aktivierung der periodischen Störkraft eingeschaltet Die periodisch wirkende Störkraft ist harmonisch und zeigt über einen Bewegungsmelder eine sinusförmige Auslenkung (Amplitude) an einem Oszilloskop der Type HAMEG HM 303-6. Die Anregungsfrequenz betrug 334,5 Hz und wurde mit dem kombinierten Frequenzgenerator und Verstärker der Type TOELLNER TOE 7741 eingestellt. Die Amplitude der Schwingung wurde am Potentiometer des Gerätes eingestellt (Stellung 2) und lag bes 1,5.

Nach der erfolgten Einstellung der periodischen Störkraft wurde ein Absperrhahn in der Zuführleitung der Schmelzphase zum Masseproportionierer 7 geöffnet und mittels Zahnradpumpe durch Variation der frequenzgeregelten Drehzahl ein Massestrom von 0,36 kg/h eingestellt. Sowohl der Pumpenkopf als auch die Zuführleitung waren außen dampfbeheizt. Der Massestrom wurde mit Hilfe eines induktiven Massedurchflusszählers 109 angezeigt bzw. als Regelgröße der Drehzahl über einen PID-Hardwareregler folgend im Automatikbetrieb geregelt. Der definierte Massestrom wurde dem Masseproportionierer 7, 8 zugefördert, wobei der Düsendurchmesser 0,3 mm betrug. Die eingestellten Strömungsverhältnisse entsprechen jenen des laminaren Strahlzerfalls mit Resonanzanregung. Unter diesen Bedingungen zeigte sich ein so genanntes "stehendes" Tropfenbild (Fig. 3) das mit einer Stroboskoplampe der Type DrelloScop 3108 R, sichtbar gemacht werden kann. Die Wellenlänge würde bei etwa 5,6 mm nach dem 7.-8. Partikel des Tropfenbildes liegen. Tatsächlich erfolgte das Eintauchen des Tropfenkollektivs nach dem 2. bis 3. Partikel des stehenden Tropfenbildes.

Die eingestellte Flüssigkeitshöhe, bei einem Strom an Erstarrungsflüssigkeit 11 von etwa 2 $m^3/h$, betrug an der Überlaufkante, das heißt an der Stelle bei welcher die Flüssigkeit unter dem Einfluss der Schwerkraft erstmals beschleunigt wird, etwa 18 mm.

Die mittels der Resonanzanregung des laminaren Strahlzerfalls erzeugten etwa masseäquivalenten Tropfen 9 wurden unter einem spitzen Winkel $\alpha$ von etwa 72°, in die kontinuierlich geführte Erstarrungsflüssigkeit 11 eingebracht. Die Erstarrungsflüssigkeit 11 war eine ethanolische $CaCl_2$-Lösung mit einer Geschwindigkeit von 0,90 m/s an der Eintropfstelle. Dies entsprach einer Re-Zahl von etwa 45.

Die Härtung der sphärischen Partikel erfolgt in diesem Beispiel durch Ionenaustausch zwischen dem in der Härterlösung vorhanden $Ca^{2+}$-Ionen und dem in der Suspension befindlichen Ammoniumion. Durch den unpolaren Anteil der Härterlösung, dies ist der Ethanol, erfolgt die Härtung nicht schlagartig, sondern wiederum etwa nach 1/3 des zurückgelegten Wegs der Härtersttrecke sukzessive von außen nach innen durch Gelieren.

Unter diesen Bedingungen konnten keramische Partikel mit einer Sphärizität von 0,991 nach der erfolgten Trocknung und Sinterung, erzeugt werden. Die Korngrößenverteilung der gesamten Fraktion ist normal verteilt und lag nach folgender Trocknung und Sinterung zwischen 0,33 bis 0,56 mm. Rund 92,7 Masse-% der hergestellten keramischen Partikel lagen in dem interessierenden Durchmesserbereich zwischen 0,36-0,5 mm. Der $d_{50}$ lag bei 0,43 mm und die sphärischen Partikel zeigten eine hohe Dichte von 6,18 kg/dm$^3$. Die Sphärizität zeigt eine relative Durchmesserabweichung von $\leq 0,3$ %.

Beispiel 2:

Als Eintropfapparat wird jener der Fig. 19 anstelle des Rinnenkanaltrichters (Fig. 5) in die Versuchsanlage geschaltet. Als Suspension wurde die unter Beispiel 1 hergestellte eingesetzt und die Stoffdaten als auch die Einstellungen des Masseproportionierers waren ident zum Beispiel 1. Es wurden Feststoffpartikel 10 auf Basis einer Keramik mit einem mittleren Durchmesser $d_{50}$ von etwa 0,43 mm mit dem 2-Phasen-Eintropfapparat (Fig. 19) hergestellt.

Als obere, leichtere und unpolare Phase der Erstarrungsflüssigkeit 11 wurde SSD-70 bei etwa 15°C mit einer Dichte von 0,788 kg/dm$^3$ eingesetzt. Dieser Phase wird die stabilisierende und formgebende Aufgabe zu Teil. Die Phasenhöhe des SSD-70 betrug 140 mm. Als härtende Phase der Erstarrungsflüssigkeit 11 wurden 3 Ma.% an Calciumchlorid in einer 93,6 Ma.%igen Ethanollösung (technische Qualität) gelöst. Diese Phase zeigt eine Dichte von 0,833 kg/dm$^3$ und wurde der SSD-70-Phase unterschichtet. Durch den hohen EtOH-Gehalt der schwereren Phase wird einerseits eine geringe Grenzflächenspannung zwischen den beiden nicht mischbaren Fluidphasen SSD-70/CaCl$_2$-EtOH von 2,7 mN/m bei 20 °C eingestellt und andererseits die chemische Härtung in der schwereren Phase verzögert. Die Fluidhöhe der schwereren Phase betrug 1,6 m. Die Oberflächenspannung der SSD-70-Phase betrug 28,6 mN/m, jene der Suspension lag bei 43,5 mN/m.

Die Abtrennung der Grünlingskugeln von der schwereren Phase der Erstarrungsflüssigkeit 11 erfolgt in einem Konus bzw. über ein Sieb 12. Unter diesen Bedingungen konnten keramische Partikel mit einer Sphärizität von 0,992 nach der erfolgten Trocknung und Sinterung, erzeugt werden. Die Korngrößenverteilung der gesamten Fraktion ist normal verteilt und lag nach folgender Trocknung und Sinterung zwischen 0,33 bis 0,56 mm. Rund 94,5 Masse-% der hergestellten keramischen Partikel lagen in dem interessierenden Durchmesserbereich zwischen 0,36-0,5 mm. Der $d_{50}$ lag bei 0,43 mm und die sphärischen Partikel zeigten eine hohe Dichte von 6,22 kg/dm$^3$ nach der Sinterung. Die Sphärizität zeigt eine relative Durchmesserabweichung von $\leq 0,3$ %.

Alle zuvor beschriebenen Ausführungsformen oder deren Teile können untereinander auch kombiniert werden.

Bezugszeichenliste

| | |
|---|---|
| 1 | Vorratsbehälter |
| 2 | fließfähige Ausgangsstoff |
| 3 | Rührorgan |
| 4 | konstantes Fluidniveau |
| 5 | Pumpe |
| 6 | Massedurchflusszähler |
| 7 | Masseproportionierer/Düse |
| 8 | elektronisch geregelter Elektromagnet |
| 9 | Tropfen |
| 10 | Feststoffpartikel |
| 11 | Erstarrungsflüssigkeit |
| 12 | mechanische Trenneinheit |
| 13 | Vorratsbehälter für Erstarrungsflüssigkeit |
| 14 | Kreiselpumpe |
| 15 | Wärmetauscher |
| | |
| 20 | Zweistoffdüse |
| 21 | Kühlmedium für Vorkühlung, Aerosol (Sprühnebel) |

| | |
|---|---|
| 30 | Einlass für Erstarrungsflüssigkeit |
| 31 | Überlaufwehr, Strömungsstörkörper, Tragflügel-Strömungsstörkörper |
| 40 | Lochplatte |
| 41 | Reservoir für Ausgangsstoff |
| 42 | Düse |
| 43 | Wandung |
| 44 | Zuleitung für fließfähigen Ausgangsstoff |
| 50 | Bewegungsbahn der Tropfen (9) |
| 60 | Rührkessel |
| 61 | Trombe bzw. Trombenform |
| 62 | Kühlmantel des Rührkessels 60 |
| 63 | Rührorgan, höhen- und drehzahlverstellbar |
| 64 | Drehzahlregler, Frequenzumformer |
| 101 | Vorratsbehälter, fließfähiger Ausgangsstoff |
| 102 | Fluidniveau |
| 103 | Pumpe |
| 104 | Masseproportionierer |
| 105 | konstantes Fluidniveau |
| 106 | Regel- bzw. Schwimmerventil |
| 107 | Druckregler |
| 108 | Druckgas |
| 109 | Massedurchflusszähler |
| 201 | Zulaufleitung, rotierender Behälter, Gleitringdichtung |
| 202 | Steigleitung, Erstarrungsflüssigkeit |
| 203 | Verteilvorrichtung Erstarrungsflüssigkeit frisch bzw. kalt |
| 204 | Ringförmig angeordnete Verteilvorrichtung Erstarrungsflüssigkeit |
| 205 | Lochöffnung der Verteilvorrichtung |
| 206 | Fluidniveau, Eintropfbereich |
| 207 | innenliegender Trichter zur Ableitung der "gebrauchten" bzw. erwärmten Erstarrungs- flüssigkeit |
| 208 | Ableitrohr gebrauchte bzw. erwärmte Erstarrungsflüssigkeit |
| 209 | Sammelkonus für sphärische Feststoffpartikel (10) |
| 210 | Ablassabsperrorgan, Kugelauslass |
| 211 | Drehbewegung, Zahnriemenscheibe Motor (vereinfacht bzw. nicht dargestellt) |
| 301. | Zuführleitung Erstarrungsflüssigkeit, geschlossenes System |
| 302. | Verteiler |
| 303. | tangential angeordnete Einleitrohre |
| 304. | ringförmig ausgebildeter Ringkanal |
| 305. | Bewegungsbahn der Feststoffpartikel (10) - schraubenförmig |
| 306. | Ableitrohr gebrauchte bzw. erwärmte Erstarrungsflüssigkeit inkl. sphärischer Feststoff- partikel. |
| 307. | Sammelkonus für sphärische Feststoffpartikel (10) und Trennvorrichtung. |
| 308. | Ablassabsperrorgan, Kugelauslass. |
| PIC | Druckregelung |
| CV | Regelventil |
| WIC | Massestromregelung |
| M | Motor |
| FIC | Durchflussmessung |

**Patentansprüche**

1. Partikel aus einem keramischen Werkstoff, **gekennzeichnet durch**

   (a) eine Sphärizität von $\geq 0{,}930$,
   (b) einen Durchmesser zwischen 20 $\mu$m und 6.000 $\mu$m, bei einer relativen Standardabweichung von $\leq 10\%$,

   wobei der keramische Werkstoff ein cerstabilisiertes Zirkonoxid mit einem $CeO_2$-Gehalt von 10 bis 30 Massen-% ist.

2. Partikel nach Anspruch 1, **gekennzeichnet durch** eine Sphärizität von größer $\geq 0{,}960$, insbesondere von $\geq 0{,}990$.

3. Partikel nach einem der Ansprüche 1 oder 2, **gekennzeichnet durch** einen Durchmesser zwischen 100 $\mu$m und 2.500 $\mu$m, jeweils bei einer relativen Standardabweichung von $\leq 5\%$, bevorzugt $\leq 4\%$, insbesondere $\leq 1\%$.

4. Partikel nach mindestens einem der Ansprüche 1 bis 3, **gekennzeichnet durch** einen Durchmesser zwischen 300 $\mu$m und 2.000 $\mu$m, bei einer relativen Standardabweichung von $\leq 3{,}5\%$.

5. Partikel nach mindestens einem der Ansprüche 1 bis 4, **gekennzeichnet durch** eine Kornrohdichte im Bereich zwischen 6,100 und 6,250 g/cm$^3$.

6. Verwendung eines Partikels nach mindestens einem der Ansprüche 1 bis 5 als Mahlkörper in Mühlen, insbesondere Hochleistungsmühlen.

**Claims**

1. A particle made of a ceramic material, **characterized by**

   (a) a sphericity of $\geq 0.930$,
   (b) a diameter between 20 $\mu$m and 6000 $\mu$m, at a relative standard deviation of $\leq 10\%$,

   wherein the ceramic material is a cerium-stabilized zirconium oxide having a $CeO_2$ content of 10 to 30% by mass.

2. The particle as claimed in claim 1, **characterized by** a sphericity of greater than $\geq 0.960$, in particular of $\geq 0.990$.

3. The particle as claimed in any of claims 1 or 2, **characterized by** a diameter between 100 $\mu$m and 2500 $\mu$m, in each case at a relative standard deviation of $\leq 5\%$, preferably $\leq 4\%$, in particular $\leq 1\%$.

4. The particle as claimed in at least one of claims 1 to 3, **characterized by** a diameter between 300 $\mu$m and 2000 $\mu$m, at a relative standard deviation of $\leq 3.5\%$.

5. The particle as claimed in at least one of claims 1 to 4, **characterized by** an apparent particle density in the range between 6100 and 6250 g/cm$^3$.

6. The use of a particle as claimed in at least one of claims 1 to 5 as milling body in mills, in particular high-performance mills.

**Revendications**

1. Particule en un matériau céramique, **caractérisée par**

   a) une sphéricité $\geq 0{,}930$ $\mu$m,
   b) un diamètre compris entre 20 $\mu$m et 6 000 $\mu$m, pour un écart-type relatif $\leq 10\%$,

   dans laquelle le matériau céramique est un oxyde de zirconium stabilisé avec du cérium ayant une teneur en $CeO_2$ de 10 à 30% en masse.

**2.** Particule selon la revendication 1, **caractérisée par** une sphéricité supérieure à $\geq 0,960$, en particulier $\geq 0,990$.

**3.** Particule selon l'une quelconque des revendications 1 ou 2, **caractérisée par** un diamètre compris entre 100 $\mu$m et 2 500 $\mu$m, à chaque fois pour un écart-type relatif $\leq 5\%$, de préférence $\leq 4\%$, en particulier $\leq 1\%$.

**4.** Particule selon au moins l'une des revendications 1 à 3, **caractérisée par** un diamètre compris entre 300 $\mu$m et 2 000 $\mu$m, pour un écart-type relatif $\leq 3,5\%$.

**5.** Particule selon au moins l'une des revendications 1 à 4, **caractérisée par** une masse volumique apparente de grains dans la plage comprise entre 6,100 et 6,250 g/cm$^3$.

**6.** Utilisation d'une particule selon l'une quelconque des revendications 1 à 5, à titre d'élément broyeur dans des broyeurs, en particulier des broyeurs de haute performance.

Massen-Proportionierer

7; 8; 9; 104

PIC 107

WIC

109

M F

103

CV

108

105
h = const

102

106

101

2

Fig. 1

Fig. 2

Fig. 3

Düsenquerschnittsfläche

$F_{Auftrieb}$ $F_{\sigma, vert.}$

$F_{\sigma, horiz.}$ $F_{Gewicht}$

Abrisslänge

$F_{Schwingung}$

Wellenlänge $\lambda$

Tropfendurchmesser $d_T$

Geschwindigkeit der
Tropfen, $u_{Tropfen}(t)$

Fig. 4

Fig. 5

Fig. 6

Fig. 7

$u_T$

$M_{Spin}$

9

11

Impact distance L

$F_{Friction}$

$u_{Fluid}$

## Fig. 8

9

## Fig. 9

$\alpha_{Trichter}$

$\alpha$

11

31

30

## Fig. 10

Fig. 11

Fig. 12

α
31
11

Fig. 13

Überströmung
Unterströmung
11
α
beweglicher Tragflügel, 31

61
60
63
α
62
11
M F 64

Fig. 14

Fig. 15

41

40

42

9

9

9

Fig. 16

44

W

41

43

42

9

9

Fig. 17

Fig. 18

Fig. 19

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4436782 A
- DE OS10019508 A1
- EP 26918 A
- EP 1136464 A2
- EP 0677325 A1
- DE 10217138 A1